# EUROPÄISCHE PATENTSCHRIFT

(11) **EP 3 773 143 B1**
(45) Veröffentlichungstag und Bekanntmachung des Hinweises auf die Patenterteilung: **27.03.2024**
(21) Anmeldenummer: 18749296.2
(22) Anmeldetag: 12.07.2018
(51) Int. Cl.: A61B 3/00

(54) **VORRICHTUNG UND VERFAHREN ZUR UNTERSUCHUNG DER RETINALEN VASKULÄREN ENDOTHELFUNKTION**
DEVICE AND METHOD FOR EXAMINING THE RETINAL VASCULAR ENDOTHELIAL FUNCTION
DISPOSITIF ET PROCÉDÉ D'ANALYSE DE LA FONCTION ENDOTHÉLIALE VASCULAIRE RÉTINIENNE

(30) Priorität: 29.03.2018 DE 102018107625
(43) Veröffentlichungstag der Anmeldung: 17.02.2021
(73) Patentinhaber: Imedos Systems GmbH, 07751 Jena (DE)
(72) Erfinder: VILSER, Walthard, 07407 Rudolstadt (DE); HAUEISEN, Jens, 07751 Jena (DE); KLEE, Sascha, 99096 Erfurt (DE); LINK, Dietmar, 98693 Ilmenau (DE); RIEMER, Thomas, 07751 Jena (DE); SKORSETZ, Martin, 07745 Jena (DE)
(74) Vertreter: Gleim Petri Patent- und Rechtsanwaltspartnerschaft mbB
(86) Internationale Anmeldenummer: PCT/DE2018/100637
(87) Internationale Veröffentlichungsnummer: WO 2019/185072

(56) Entgegenhaltungen:
- WO-A1-99/29229
- WO-A2-2013/120085
- ANDREW O'BRIEN ET AL: "Imaging system to assess objectively the optical density of the macular pigment in vivo", APPLIED OPTICS, Bd. 52, Nr. 25, 23. August 2013 (2013-08-23), Seite 6201, XP055537429, US ISSN: 1559-128X, DOI: 10.1364/AO.52.006201
- GERHARD GARHOFER ET AL: "Use of the retinal vessel analyzer in ocular blood flow research", ACTA OPHTHALMOLOGICA: THE OPHTHALMOLOGICAL JOURNAL OF THE NORDIC COUNTRIES, Bd. 88, Nr. 7, 1. November 2010 (2010-11-01), Seiten 717-722, XP055537435, Denmark ISSN: 1755-375X, DOI: 10.1111/j.1755-3768.2009.01587.x

## Beschreibung

Das Anwendungsgebiet der Erfindung betrifft die gesamte vaskuläre Medizin, z. B. die Augenheilkunde, Neurologie, Kardiologie, Nephrologie, Diabetologie und Hypertensiologie.

Aus Studien ist bekannt, dass mikrovaskuläre Gefäßveränderungen häufig systemischer Art sind, d. h. dass sie in den Gefäßen, insbesondere den Gefäßen der Mikrozirkulation aller Organe des menschlichen und tierischen Körpers, ähnlich auftreten und je nach Organ zu unterschiedlichen Ausprägungen von kardiovaskulären Erkrankungen, wie Atherosklerose, Arteriosklerose, Herzinsuffizienz, Niereninsuffizienz, Augenerkrankungen wie z. B. Retinopathien und Glaukom, zerebro-vaskulären Erkrankungen, wie z. B. vaskulärer Demenz, führen und letztlich kardiovaskuläre Ereignisse, wie Herzinfarkt und Schlaganfall, auslösen können bzw. deren Prädiktoren sind.

Das Auge als einzigartiges optisches Fenster zur Mikrozirkulation erlaubt es, die Netzhautgefäße als Spiegelbild der Gefäße und deren Funktionen in nicht zugänglichen Bereichen der anderen Organe des Körpers zu untersuchen. Ein bevorzugtes Anwendungsgebiet der Erfindung ist die Ergänzung der Gefäßdiagnostik der großen Gefäße um die Gefäßdiagnostik der Mikrozirkulation und insbesondere die Funktionsdiagnostik der vaskulären Endothelfunktion bzw. der vaskulären Dysregulation.

Derzeit werden in der Augenheilkunde vor allem bildgebende Verfahren für klinische Fragestellungen eingesetzt, die strukturelle bzw. morphologische Veränderungen am Auge, insbesondere am Augenhintergrund (in der Netzhaut) untersuchen. Dazu gehören konventionelle Funduskameras, OCT und Laserscanner. Gefäßuntersuchungen mit der statischen Gefäßanalyse, wie z. B. mit dem VesselMap der Firma Imedos, beginnen im klinischen Alltag zur mikrovaskulären Risikostratifizierung und Therapiekontrolle einzudringen.

Funktionelle Untersuchungen der Netzhautgefäße haben bisher vorwiegend nur Anwendung in der Forschung gefunden, wie z. B. Vorrichtungen und Verfahren zur Messung von Blutgeschwindigkeit und Gefäßdurchmessern auf Indikatorbasis, Doppler- oder OCT-Basis sowie Systeme zur dynamischen Gefäßanalyse. Die Einsatzgebiete der Systeme auf Doppler- oder OCT-Basis liefern Aussagen, die kaum Bedeutung außerhalb der Augenheilkunde erlangten und keine Funktionsdiagnostik der Autoregulation bzw. der Endothelfunktion ermöglichen.

Die dynamische Gefäßanalyse ermöglicht die Untersuchung verschiedener Autoregulationsmechanismen auf der Basis von kontinuierlichen Messungen der Gefäßdurchmesser über die Zeit und entlang des Ortes für die großen Arterien und Venen der Mikrozirkulation. Die Netzhautgefäße bzw. die Mikrozirkulation werden während der Messung und Aufzeichnung der Gefäßdurchmesser stimuliert oder provoziert und antworten dementsprechend mit einer Konstriktion oder Dilatation, die die Gefäßantwort der jeweiligen durch die Stimulationsart oder Provokationsart angesprochenen retinalen Autoregulation und deren Funktionsfähigkeit beschreibt.

Mit derartigen Verfahren zur Stimulation oder Provokation kann man verschiedene Autoregulationsmechanismen der Mikrozirkulation untersuchen. Eine der Autoregulationsmechanismen ist die flussinduzierte Autoregulation. Die Stimulation wird dabei mit Flickerlicht (rhythmische Unterbrechung des Flickerlichtes mit einer definierten Frequenz) vorgenommen, wobei wegen der technischen Unzulänglichkeiten, dass das Messlicht zugleich als Flickerlicht verwendet wird, die Parameter für das Messlicht und das Flickerlicht nicht unabhängig voneinander einstellbar sind.

Das den Stand der Technik verkörpernde System der dynamischen Gefäßanalyse ist der Retinal Vessel Analyzer (RVA) bzw. der Dynamic Vessel Analyzer (DVA) der Firma Imedos (Garhofer, G., Bek, T., Böhm, A.G., Gherghel, D., Grunwald, J., Jeppesen, P., Kergoat, H., Kotliar, K., Lanzl, I., Lovasik, J.V., Nagel, E., Vilser, W., Orgul, S., Schmetterer, L.: "Use of the retinal vessel analyzer in ocular blood flow research". Acta Ophthalmologica 2010: 88: Seiten 717-722.) Die verwendete Standardprovokation im RVA/DVA ist Flickerlicht, das mit einer Frequenz von 12,5 Hz arbeitet. Weißes Halogenlampenlicht im kontinuierlichen Beleuchtungsstrahlengang einer konventionellen Funduskamera wird mit einem Bandpassfilter spektral in grünes Messlicht modifiziert und während der festen Stimulationsphasen von gewöhnlich 20 s durch einen elektrooptischen Shutter rhythmisch unterbrochen. Die Untersuchung besteht aus 3 Phasen, wobei die erste Phase (Baseline-Phase BP) den Baseline-Gefäßdurchmesser aufzeichnet und als Referenz zur Berechnung prozentualer Gefäßantworten verwendet wird. Die zweite Phase ist die Stimulationsphase (SP), in der die Gefäßantwort auf Flickerlicht aufgezeichnet wird. Die dritte Phase soll als Nachphase (NP) bezeichnet werden, da in dieser Phase der Gefäßdurchmesser wieder zur Baseline zurückkehrt. Die zweite und dritte Phase werden im Wechsel dreimal wiederholt und die Gefäßantworten werden anschließend für eine Mittelwertbildung überlagert und bezüglich der maximalen Dilatation (Flickerdilatationsmaximum FDₘₐₓ) und anschließenden Konstriktion ausgewertet.

Diese Messungen sind im Stand der Technik, gebildet durch das vorgenannte DVA, auf die großen Gefäße der Mikrozirkulation zwischen 60 und 300 µm beschränkt.

Die ausgewerteten Parameter der Gefäße, wie das Flickerdilatationsmaximum FDₘₐₓ, sowie andere ableitbare Parameter werden als Biomarker für die funktionsdiagnostische Untersuchung der mikrovaskulären Endothelfunktion interpretiert. Fälschlicherweise werden von einigen Autoren die Parameter der Gefäßantwort auch als Parameter der neurovaskulären Kopplung NVK bezeichnet und interpretiert. Es gilt aber als erwiesen, dass die neurovaskuläre Kopplung NVK zwar den Ausgangsreiz darstellt, das FDₘₐₓ der Gefäßantwort der großen Gefäße aber die Funktion der vaskulären Endothelzellen beschreibt und damit die vaskuläre Endothelfunktion charakterisiert und sich die vaskuläre Endothelfunktion bzw. die endotheliale Dysfunktion bzw. die vaskuläre Dysregulation untersuchen lässt.

Die Untersuchungstechnik des vorgenannten DVA ist zu starr, erlaubt keine Erweiterung der medizinischen Fragestellungen durch Änderung der Art und Form der Lichtstimulation sowie auch keine Optimierung für ausreichend stabile und hohe Dilatationsantworten. Zudem verursacht die unflexible Beleuchtung hohe Herstellungskosten, zusätzliches Streu- und Reflexionslicht, insbesondere auf den Gefäßen, und reduziert damit die Genauigkeit und Sicherheit der medizinischen Aussage sowie die Einsatzbreite in der Forschung und Klinik. Der Herstellungspreis ist hoch, die Untersuchung für den Patienten belastend und die Reproduzierbarkeit der Parameter der Gefäße für die individuelle Untersuchung ist noch nicht ausreichend zufriedenstellend. Das vorgenannte DVA benutzt einen monochromatischen digitalen Bildsensor und nur einen spektralen Wellenlängenbereich im grünen Licht, der zugleich Messlicht und Stimulationslicht erzeugt. Durch den elektrooptischen Shutter entsteht eine prinzipbedingte Abhängigkeit der Modulation von Mess- und Stimulationslicht, die dazu führt, dass die Anordnung entweder nur für die Messungen oder nur für die Stimulation optimiert werden kann. Die Optimierungskriterien zwischen beiden Beleuchtungsarten sind aber sehr unterschiedlich. Weiterhin treten subjektive untersucherbedingte Fehler auf und die Untersuchung stellt hohe Anforderungen an den Untersucher im Umgang mit dem Patient und dem vorgenannten DVA. Im Weiteren ist mit der unflexiblen und starren technischen Lösung der Erzeugung des Stimulationslichtes beim DVA eine nur begrenzte Einsatzbreite des DVA in der Funktionsdiagnostik mit Flickerlicht in Forschung und Klinik gegeben.

In "Imaging system to assess objectively the optical density oft he macular pigment in vivo" von O'brien et al. wird eine Vorrichtung offenbart, die zur Bestimmung der optischen Dichte der im Bereich der Makula vorhandenen Pigmente geeignet ist. Die Vorrichtung weist einen ersten Beleuchtungsstrahlengang zur Messung und einen zweiten Beleuchtungsstrahlengang zur Kalibrierung sowie eine Funduskamera auf, die die Netzhaut abbildet. Im zweiten Beleuchtungsstrahlengang ist zur Kalibrierung eine Fixationsmarke vorgesehen. Die Gefäße der Netzhaut sorgen dafür, dass die Genauigkeit der Messung abnimmt und werden daher mithilfe eines Algorithmus ausgeblendet. Die Vorrichtung weist außerdem keine Makulablende auf.

Im Weiteren wird mit der WO 2005/094668 A1 eine Vorrichtung zur photometrischen Messung der Gefäßdurchmesser kleinerer Gefäße beschrieben. Die offenbarte technische Lösung ermöglicht die Messung von Gefäßdurchmessern im Bereich der Arteriolen und Venolen, sofern die Gefäße im Fundusbild als Gefäße selektierbar sind. Dazu werden zwei verschiedene spektrale Wellenlängenbereiche einer Farbkamera benutzt. Das erhöht die Lichtbelastung der Netzhaut beträchtlich. Ein weiterer wesentlicher Nachteil der offenbarten Lösungen besteht aber ebenfalls in der starren beleuchtungsseitigen Anordnung eines Lichtmodulators im gemeinsamen beleuchtungsseitigen Strahlengang der beiden Farbkanäle der Farbkamera, der ebenfalls nur die zeitliche Modulation flexibel gestalten lässt und die Einsatzbreite und Adaptivität wesentlich einschränkt. Letztendlich weist die Vorrichtung der vorgenannten WO 2005/094668 A1 die gleichen Nachteile auf wie das vorgenannte DVA, bis auf den Vorteil, dass auch an kleinen Netzhautgefäßen, die aber deutlich größer als Kapillaren sind, gemessen werden kann.

Eine weitere technische Lösung zur Erfassung der kapillaren "Perfusion" wird mit dem Artikel von Vilser et. AI von 2008 (Vilser, W., Nagel, E., Seifert, B.U., Riemer, T., Weisensee, J., Hammer, M: "Quantitative assessment of optic nerve head pallor". Physiological Measurement 29 (2008), Seiten 451-457) beschrieben. Über einen Dualbandpassfilter im Beleuchtungsstrahlengang einer konventionellen Funduskamera werden zwei Spektralbereiche im roten und grünen Spektralbereich des weißen Beleuchtungslichtes selektiert und einem roten und einem grünen Farbkanal eines 3-Chip-Farbbildsensors derart zugeordnet, dass beide selektierten beleuchtungsseitigen Wellenlängen jeweils getrennt voneinander durch die beiden zugeordneten roten und grünen Farbkanäle des Farbbildsensors empfangen werden. Aus den von den Pixeln der beiden Farbkanäle (rot und grün) detektierten Farbintensitäten, die jeweils einem gleichen Funduspunkt zugeordnet werden können, werden Quotienten gebildet und dem Fundusort wieder zugeordnet. Das so erzeugte Quotientenbild wird dann bezüglich der kapillaren Perfusion auf dem Sehnervenkopf ausgewertet.

Mit dieser Methode kann man zwar nicht die Perfusion des Sehnervenkopfes darstellen, wenn man unter Perfusion den kapillaren Blutfluss versteht, aber man hat ein Maß für das Blutvolumen und damit für die kapillaren Gefäßdurchmesser und die Kapillarisierung der betrachteten Gewebevolumina. Der Nachteil dieser Methode ist, dass sie nicht wie in dem vorgenannten Artikel beschrieben funktionelle Aussagen über die Regelung der kapillaren Perfusion liefern kann.

Es ist die Aufgabe der Erfindung, ein Verfahren zu finden, mit dem eine Untersuchung der retinalen vaskulären Endothelfunktion für den Patienten weniger belastend ist.

Es ist auch die Aufgabe der Erfindung, eine zur Durchführung des Verfahrens geeignete Vorrichtung zu finden.

Für eine erfindungsgemäße Vorrichtung ist es erfindungswesentlich, dass in deren Beleuchtungsstrahlengang eine Makulablende in einer zum Fundus konjugierten Ebene angeordnet ist.

Der Stand der Technik geht davon aus, dass die Flickerstimulation im Bereich der Makula entscheidend für die Dilatation der großen Gefäße der Netzhaut ist. Erfindungsgemäß wird gerade der Bereich der Makula beleuchtungsseitig durch eine Makulablende abdeckt und damit nicht mit Flickerlicht stimuliert, was in überraschender Weise die Reaktion der großen Gefäße (Gefäßantwort), die außerhalb der Makula mit Mess- und Flickerlicht beaufschlagt werden, nicht beeinträchtigt.

Aus der Verwendung der Makulablende ergeben sich folgende Vorteile:
1. Sie reduziert drastisch die Lichtbelastung des Patienten, da die lichtempfindliche Makula während der Untersuchungen abgedeckt bleibt.
2. Alternativ kann mehr Licht im blendenfreien Stimulations- und Messbereich, das heißt in dem nicht von der Makulablende abgedeckten Leuchtfeld auf dem Fundus, eingesetzt werden, so dass der Fundus außerhalb der Makula mit einer höheren Lichtintensität beleuchtet werden kann, womit sich ein verbessertes Signal-RauschVerhältnis ergibt, der Stimulationseffekt verbessert wird, die Dilatation erhöht und die Bildqualität im Messbereich um die Makulablende herum deutlich verbessert wird.
3. Der Patient kann bei abgedunkelter Makula durch die Makulablende eine zur Innenfixation der Foveola angebotene Fixationsmarke aufgrund des großen Helligkeitskontrastes besser finden und im Blick behalten. Die Untersuchung störende unruhige Augenbewegungen werden deutlich reduziert, was die Qualität der Messungen erheblich verbessert.
4. Ein weiterer Vorteil ist, dass mit der Makulablende, mittig angeordnet, Reflexe an der Ophthalmoskoplinse der Funduskamera vollständig ausgeblendet werden können. Auch wird abbildungswirksames Streulicht gerade aus dem zentralen Bereich der überlagerten beleuchtungsseitigen und abbildungsseitigen Strahlenbündel, was als störende und kontrastmindernde Aufhellung im Bild des Fundus erscheint, deutlich reduziert.
5. Damit kann im Beleuchtungsstrahlengang der Funduskamera auf aufwendige und teure Optikeinheiten, die mit Antireflexmaßnahmen verbunden sind, verzichtet werden. Die Baulänge und Kosten für die Entwicklung und Herstellung der Funduskamera können vergleichsweise reduziert werden. Im Weiteren wird der Lichtstrom in das Auge reduziert
6. Die Makulablende und ggf. erfindungsgemäß weitere im Beleuchtungsstrahlengang eingestellte Blenden stellen innerhalb des Leuchtfeldes Feldbereiche für die Nutzung weiterer Strahlengänge oder Strahlenbündel zur Verfügung, die zur exakten automatisierungsfähigen Einstellung der Funduskamera auf das Auge und damit zur Ausschaltung von Fehlerquellen dienen und vorteilhafterweise direkt mit dem digitalen Bildsensor der Funduskamera, ohne zusätzliche Detektoren, nutzbar sind.

Vorteilhaft befindet sich ein teiltransparenter Steg an der Makulablende. Er dient einerseits als mechanische Halterung der Makulablende und andererseits als eine weitere im Beleuchtungsstrahlengang eingestellte Blende zur Abdeckung des Sehnervenkopfes (Papille), um den Dynamikbereich des digitalen Bildsensors der Funduskamera zu optimieren und eine Überstrahlung durch sehr helle Flächenbereiche des Sehnervenkopfes zu vermeiden.

Vorteilhaft ist die Beleuchtungseinheit der Funduskamera eine adaptive, geometrisch strukturierbare Beleuchtungseinheit, z. B. in Form einer ringförmigen LED-Anordnung. Damit können das Flickerlicht und Messlicht sowohl zeitlich, geometrisch, spektral und bezüglich der zeitlichen Intensitätssteuerung voneinander erzeugt werden.

Die Aufgabe der Erfindung wird für eine Vorrichtung zur Untersuchung der retinalen vaskulären Endothelfunktion der Gefäße der Netzhaut am Fundus eines Auges, enthaltend eine Funduskamera mit einem Beobachtungsstrahlengang mit einem digitalen Bildsensor zur Aufnahme von Bildfolgen von Bildern von Bereichen des Fundus, auf den der Beobachtungsstrahlengang der Funduskamera scharf eingestellt ist, und mit einem Beleuchtungsstrahlengang, in dem in einer zu der Augenpupille des Auges konjugierten Ebene eine Beleuchtungseinheit angeordnet ist, zur Beleuchtung des Fundus mit einem Mess- und einem Flickerlicht innerhalb eines Leuchtfeldes auf dem Fundus, das durch die Abbildung einer Feldblende begrenzt ist, die in einer zum Fundus konjugierten Ebene im Beleuchtungsstrahlengang steht, dadurch gelöst, dass in der zum Fundus konjugierten Ebene im Beleuchtungsstrahlengang wenigstens eine Makulablende angeordnet ist, an der sich jeweils eine Fixationsmarke befindet, so dass eine der wenigstens einen Makulablende die Makula auf dem Fundus abdeckt, wenn das Auge auf die Fixationsmarke der einen Makulablende fixiert.

Vorteilhaft ist die Makulablende eine mechanische Blende oder eine optoelektronische Blende, z. B ein Transmissionsdisplay.

Es ist von Vorteil, wenn genau eine Makulablende vorhanden ist, die in der zum Fundus konjugierten Ebene im Beleuchtungsstrahlengang angeordnet ist, und die Fixationsmarke eine punktförmige Öffnung in einem Flächenmittelpunkt der Makulablende ist.

Ferner grenzt an die Makulablende bevorzugt ein zu deren Flächenmittelpunkt radial ausgerichteter teiltransparenter Steg an, mit dem die Papille am Fundus überdeckt werden kann, sodass die Strahlungsintensität einer Abbildung der Papille auf einen Dynamikbereich des digitalen Bildsensors angepasst werden kann, der für die Strahlungsintensität einer Abbildung der die Papille umgebenden Bereiche des Fundus ausgelegt ist.

Vorteilhaft ist eine weitere teiltransparente Blende mit einstellbarer Transparenz zur Abdeckung der Papille vorhanden.

Vorzugsweise ist die Makulablende in der zum Fundus konjugierten Ebene im Beleuchtungsstrahlengang verschiebbar, wobei deren Flächenmittelpunkt innerhalb der Feldblende liegend verbleibt, womit unterschiedliche ausgewählte Bereiche des Fundus von dem Leuchtfeld ausgeleuchtet werden und Bildfolgen von Bildern der unterschiedlichen ausgewählten Bereichen des Fundus aufgenommen werden können.

Bevorzugt ist genau eine Makulablende an der Feldblende so ausgebildet, dass die Fixationsmarke an einen inneren Rand angrenzend innerhalb der Feldblende liegt, und die Feldblende ist um eine optische Achse des Beleuchtungsstrahlenganges drehbar, womit unterschiedliche ausgewählte Bereiche des Fundus von dem Leuchtfeld ausgeleuchtet werden und Bildfolgen von Bildern der unterschiedlichen ausgewählten Bereiche des Fundus aufgenommen werden können.

Eine weitere bevorzugte Variante besteht darin, dass genau vier Makulablenden an der Feldblende paarweise diagonal gegenüberliegend so ausgebildet sind, dass die jeweilige Fixationsmarke an einen inneren Rand angrenzend innerhalb der Feldblende liegt, womit unterschiedliche vorbestimmte Bereiche des Fundus von dem Leuchtfeld ausgeleuchtet werden und alternativ Bildfolgen von Bildern der unterschiedlichen vorbestimmten Bereiche des Fundus aufgenommen werden können.

Es ist von Vorteil, wenn die Beleuchtungseinheit durch eine adaptive, strukturierbare Anordnung von Lichtquellen in der zur Augenpupille konjugierten Ebene, die örtlich, spektral und zeitlich beliebig und voneinander getrennt zu- und abgeschaltet und / oder in ihrer Intensität moduliert werden können, gebildet ist. Damit ist es möglich, verschiedenste Beleuchtungsstrukturen zu realisieren. Über die Auswahl und Ansteuerung einzelner Lichtquellen wird durch die jeweils aktiven (leuchtenden) Lichtquellen die Geometrie, z. B. ein Ring, eine Halbring oder Ringsegmente und die Dimension der Beleuchtungsstruktur, z. B. durch einen Innendurchmesser dᵢ und einen Außendurchmesser dₐ, vorgegeben. Durch eine Ansteuerung spektral unterschiedlicher Lichtquellen können für verschiedene Spektralbereiche verschiedene zeitlich und örtlich verschiedene Beleuchtungsstrukturen gebildet werden. Eine so gestaltete Beleuchtungseinheit gestaltet eine erfindungsgemäße Vorrichtung vorteilhaft aus. Eine Vorrichtung gemäß dem Stand der Technik miteiner erfindungsgemäßen Beleuchtungseinheit löst jedoch auch für sich allein, ohne eine Makulablende die Aufgabe der Erfindung.

Vorzugsweise weist eine Abbildung der im Beleuchtungsstrahlengang stehenden Feldblende auf dem digitalen Bildsensor einen kleineren Querschnitt auf als eine Empfangsfläche des digitalen Bildsensors, und die Helligkeitsverteilung in einem sich bildenden Differenzbereich wird genutzt, um eine Streulichtverteilung zu ermitteln, mit der die Bilder der Bildfolgen korrigiert werden können.

Die Aufgabe wird ferner für ein Verfahren zur Untersuchung der retinalen vaskulären Endothelfunktion der Gefäße der Netzhaut am Fundus eines Auges, enthaltend die Verfahrensschritte der Einstellung einer Funduskamera auf das Auge, der Erzeugung von Messlicht zur Beleuchtung der Gefäße der Netzhaut am Fundus und von Flickerlicht zur Stimulation der Gefäße der Netzhaut am Fundus während einer Stimulationsphase, der Erzeugung einer Bildfolge von Bildern eines Bereiches des Fundus während einer Baseline-Phase, wenigstens einer Stimulationsphase und wenigstens einer Nachphase, der Messung der Gefäßdurchmesser selektierter Gefäßsegmente der Gefäße der Netzhaut in den Bildern der erzeugten Bildfolge abhängig von Ort und Zeit, der Bewegungskorrektur für die gemessenen Gefäßsegmente, bei der jedes Gefäßsegment einem Ort am Fundus bewegungskorrigiert zugeordnet wird, der Bildung von Durchmessersignalen, die die gemessenen Gefäßdurchmesser als Funktion der Zeit und des Ortes des jeweils selektierten Gefäßsegmentes darstellen und des Ableitens von Gefäßparametern aus den Durchmessersignalen, die jeweils die Endothelfunktion des jeweils selektierten Gefäßsegmentes beschreiben, entweder dadurch gelöst, dass die Beleuchtung und Stimulation der Makula am Fundus verhindert wird, indem wenigstens eine Makulablende, an der sich eine Fixationsmarke befindet, auf dem Fundus scharf abgebildet wird und das Auge durch Fixierung auf die Fixationsmarke einer der wenigstens einen Makulablende so zu der einen Makulablende ausgerichtet wird, dass die Makula durch eine Abbildung der einen Makulablende überdeckt ist, oder dadurch gelöst, dass der Fundus mit einer adaptiv strukturierbaren Anordnung von Lichtquellen beleuchtet wird, die mit einer gebildeten Beleuchtungsstruktur an die jeweilige Öffnung der Augenpupille und andere gegebene Bedingungen anpassbar ist und über die unabhängig voneinander die Messlichtparameter und Flickerlichtparameter eingestellt werden können.

Es ist von Vorteil, wenn nacheinander Bildfolgen von Bildern ausgewählter unterschiedlicher Bereiche des Fundus aufgenommen werden, jeweils nachdem genau eine auf den Fundus abgebildete Makulablende in eine andere Lage verschoben wurde, so dass deren Abbildung auf dem Fundus verschoben wurde und das Auge die Fixationsmarke fixierend gefolgt ist.

Ferner ist es vorteilhaft, wenn die Lageparameter der Lagen der Makulablende gespeichert werden und für Wiederholungs- und Folgemessungen wieder aufrufbar und einstellbar sind.

Vorzugsweise werden alternativ Bildfolgen von vorgegebenen unterschiedlichen Bereichen des Fundus aufgenommen, während vier Makulablenden, die diagonal gegenüberliegend angeordnet sind, auf den Fundus abgebildet werden und jeweils das Auge nacheinander eine andere der Fixationsmarken fixiert hat.

Die Dauer der Baseline-Phase, der Stimulationsphase und der Nachphase und die Parameter des Messlichtes und des Flickerlichtes werden bevorzugt unabhängig voneinander eingestellt und einem Patienten und einem Untersuchungsprogramm zugeordnet gespeichert, so dass sie für Wiederholungs- und Vergleichsuntersuchungen als Parametersatz aufgerufen und wieder eingestellt werden können.

Das Flickerdilatationsmaximum wird vorteilhaft als einer der abgeleiteten Gefäßparameter ermittelt und in einem Messprotokoll gemeinsam mit einer grafischen Darstellung von gemittelten Durchmessersignalen ausgegeben, die jeweils für die selektierten arteriellen und für die selektierten venösen Gefäßsegmente aus den dafür gebildeten Durchmessersignalen gebildet werden.

Es ist dabei von Vorteil, wenn das Flickerdilatationsmaximum in einem Mappingbild als Funktionsimaging der Endothelfunktion dem jeweils zugehörigen Gefäßsegment farblich kodiert zugeordnet wird, wobei eine fehlende Gefäßdilatation oder eine Gefäßdilatation unterhalb eines vorgegebenen Schwellwertes mit rot und eine Gefäßdilatation oberhalb eines Schwellwertes, die einer gesunden Gefäßfunktion entspricht, mit grün auf dem zugehörigen Gefäßsegment markiert wird und das Mappingbild als grafisches Untersuchungsergebnis ausgegeben wird.

Die Erfindung wird anhand nachfolgender Ausführungsbeispiele unter Zuhilfenahme von Zeichnungen näher erläutert. Hierzu zeigen:
Fig. 1: ein Blockschaltbild für eine erfindungsgemäße Vorrichtung,
Fig. 2: ein Optikschema für eine erfindungsgemäße Funduskamera,
Fig. 3: einen durch das Bild einer Feldblende begrenzt ausgeleuchteten Bereich (Leuchtfeld) des Fundus eines Auges,
Fig. 4a: eine auf dem Fundus mittig abgebildete Makulablende,
Fig. 4b: eine auf dem Fundus außermittig abgebildete Makulablende,
Fig. 5a: eine auf dem Fundus abgebildete, an der Feldblende ausgebildete Makulablende,
Fig. 5b: vier auf dem Fundus abgebildete, an der Feldblende ausgebildete Makulablenden,

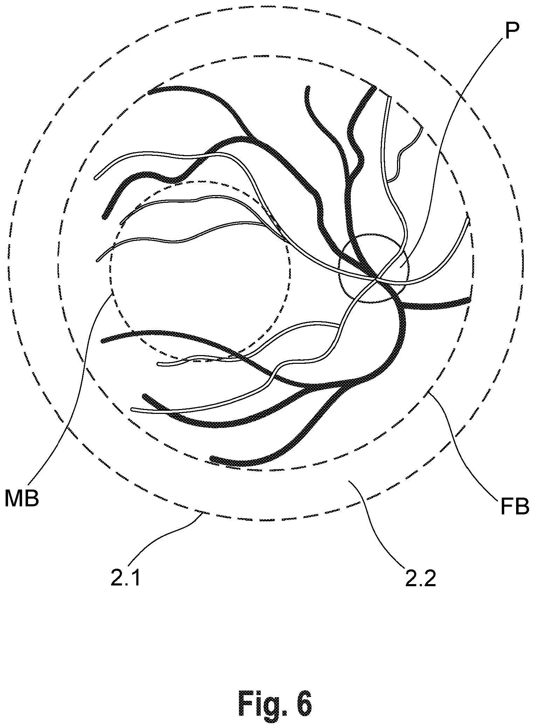

Fig. 6: die Abbildungen der Feldblende sowie der Makulablende auf der Empfangsfläche des digitalen Bildsensors,
Fig. 7a: ein erstes Beispiel für die Ausführung einer Beleuchtungseinheit als adaptive, strukturierbare Anordnung von Lichtquellen und
Fig. 7b: ein zweites Beispiel für die Ausführung einer Beleuchtungseinheit als adaptive, strukturierbare Anordnung von Lichtquellen.

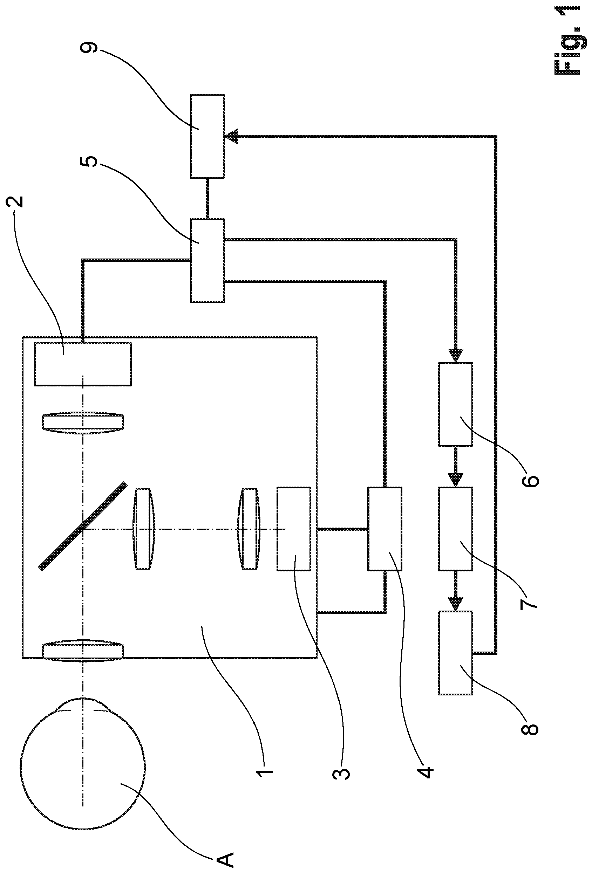

Eine Ausführung einer erfindungsgemäßen Vorrichtung ist als Blockschaltbild in Fig. 1 dargestellt. Gleich einer aus dem Stand der Technik bekannten Vorrichtung enthält diese eine Funduskamera 1 mit einem digitalen Bildsensor 2 und einer Beleuchtungseinheit 3, eine Steuereinheit 4, eine Daten- und Bildverarbeitungseinheit 5, eine Einheit zur Erzeugung von Durchmessersignalen 6, eine Signalanalyseeinheit 7, eine Ergebnis- und Präsentationseinheit 8 und eine Ein- und Ausgabeeinheit 9. Die Vorrichtung unterscheidet sich von einer gattungsgleichen Vorrichtung des Standes der Technik wesentlich in der Ausführung der Funduskamera 1 und vorteilhaft in der Ausführung der Beleuchtungseinheit 3.

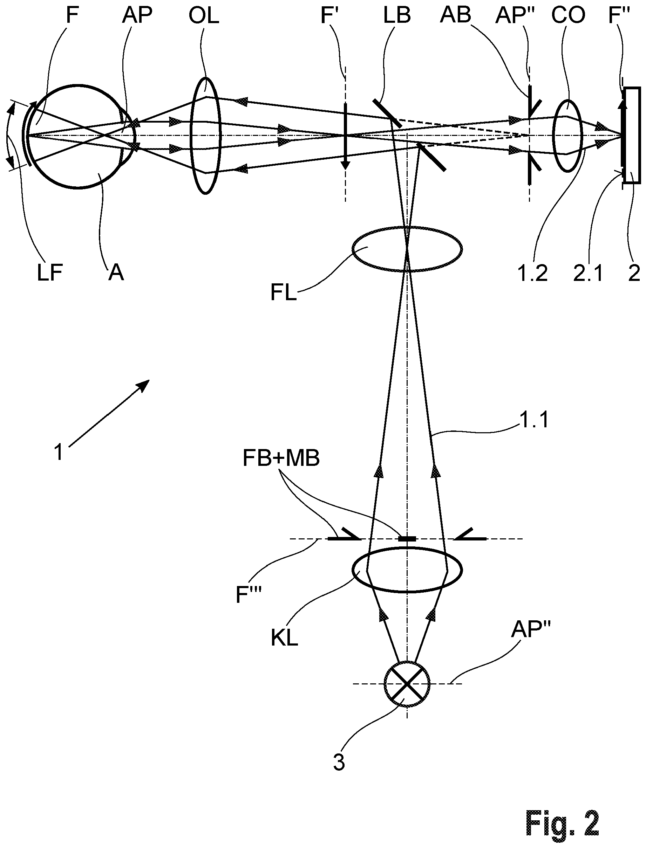

In Fig. 2 ist ein Optikschema einer Funduskamera 1 dargestellt. Sie enthält einen Beleuchtungsstrahlengang 1.1 und einen Beobachtungsstrahlengang 1.2 (Abbildungsstrahlengang).

Der Beobachtungsstrahlengang 1.2 hat im einfachsten Fall zwei Linsen, eine Ophthalmoskoplinse OL und eine Objektivlinse CO, über die der Fundus F des Auges A, auf den die Funduskamera 1 scharf eingestellt wurde, in eine zum Fundus F konjugierte Ebene F" auf einer Empfangsfläche 2.1 des digitalen Bildsensors 2 abgebildet wird.

Der Beleuchtungsstrahlengang 1.1 wird über eine Lochblende LB in den Beobachtungsstrahlengang 1.2 eingekoppelt und enthält im einfachsten Fall eine Kollimatorlinse KL und eine Feldlinse FL. Die Beleuchtungseinheit 3 steht in einer zur Augenpupille AP konjugierten Ebene im Beleuchtungsstrahlengang 1.1 und wird in die Augenpupille AP abgebildet. Die Feldblende FB, die in einer zum Fundus F konjugierten Ebene F‴ im Beleuchtungsstrahlengang 1.1 steht, wird scharf auf dem Fundus F und auf der Empfangsfläche 2.1 des digitalen Bildsensors 2 abgebildet.

Es ist erfindungswesentlich, dass im Beleuchtungsstrahlengang 1.1 zusätzlich eine Makulablende MB vorhanden ist. Die Makulablende MB ist bevorzugt in einer gleichen Ebene wie die Feldblende FB angeordnet, beide können aber auch in unterschiedlichen zueinander konjugierten Ebenen angeordnet sein. Dabei kann die Makulablende MB innerhalb der Feldblende FB, die typischerweise durch einen Ring gebildet ist, fest oder verschiebbar angeordnet sein. Verschiedene vorteilhafte Ausführungen werden anhand von Ausführungsbeispielen erläutert.

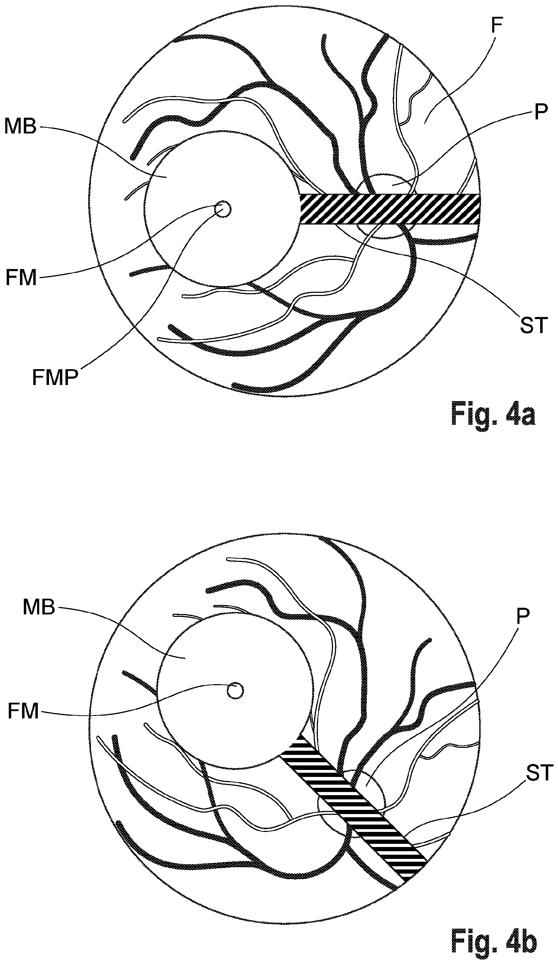

Gemäß einem ersten Ausführungsbeispiel, dargestellt in **Fig. 4a****,** ist die Makulablende MB kreisförmig, zentral angeordnet, dass heißt ihr Flächenmittelpunkt FMP liegt auf der optischen Achse des Beleuchtungsstrahlenganges 1.1, und wird mit einem Feldwinkel von 15° auf den Fundus F abgebildet. Die Makulablende MB enthält zur Innenfixation um ihren Flächenmittelpunkt FMP eine kleine Öffnung als Fixationsmarke FM. Alternativ können anstelle der Öffnung als Fixationsmarke FM kleine Lichtquellen z. B. LEDs, strukturiert als Punkte, Ringe oder Kreuze, zeitlich, geometrisch und spektral unterschiedlich über die Steuereinheit 4 ansteuerbar als leuchtende Fixationsmarken FM eingespiegelt werden. Damit können einerseits durch die geometrische Strukturierung unterschiedliche Einstellungen des Fundus F zur Makulablende MB hergestellt werden. Andererseits können blinkende Fixationsmarken FM oder Farbänderungen die Aufmerksamkeit des Patienten erhöhen oder an die Sehfähigkeit des Patientenauges angepasst werden. Das zu untersuchende Auge A könnte auch durch eine Außenfixation fixiert werden, sodass es keiner Fixationsmarke FM innerhalb der Funduskamera 1 bedarf. Insbesondere wenn das zu untersuchende Auge A so sehschwach ist, dass es nicht auf eine Fixationsmarke FM fixieren kann, ist es sinnvoll, dass eine auf die Lage der Makulablende MB abgestimmt positionierte Fixationsmarke FM außerhalb der Funduskamera 1 dem nicht zu untersuchenden Auge A angeboten wird.

In **Fig. 4b** ist die Makulablende MB in einer gegenüber der Fig. 4a verschobenen Position dargestellt. Entsprechend liegt jetzt ein anderer Bereich des Fundus F innerhalb des Leuchtfeldes LF, welches durch die Abbildung der Feldblende FB auf dem Fundus F begrenzt wird.

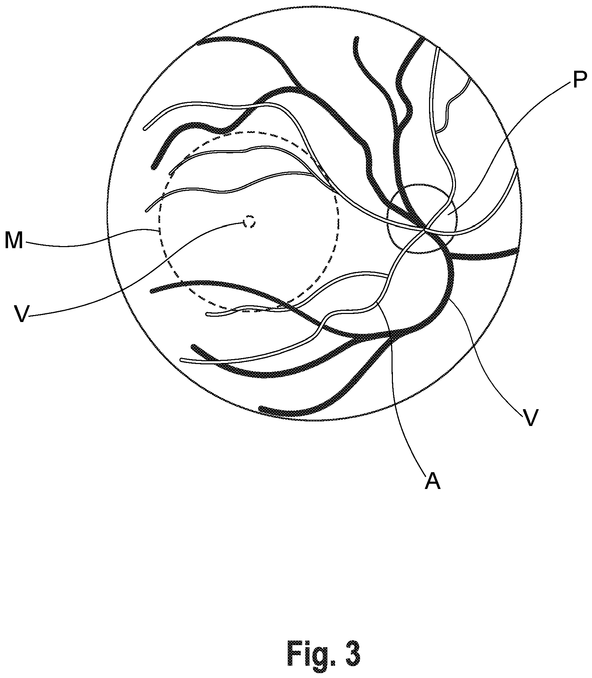

In **Fig. 3** ist für ein einfaches Verständnis eine Struktur des Fundus F eines Auges A schematisch dargestellt. Gezeigt sind neben großen Arterien Av (hellgrau) und großen Venen Vv (dunkelgrau) die Makula M, der Bereich mit der größten Dichte von Sehzellen (gelber Fleck) und damit auch der lichtempfindlichste Bereich des Fundus F, die Foveola V, die Sehgrube im Zentrum der Makula M, mit der das Auge A auf eine Fixationsmarke FM fixiert, und die Papille P (Sehnervenkopf, blinder Fleck), die Austrittsstelle des Sehnervs aus der Hülle des Augapfels, die vergleichsweise wenig durchblutet ist.

Die Feldblende FB und die Makulablende MB sind gemeinsam axial im Beleuchtungsstrahlengang 1.1 verschiebbar, so dass die Makulablende MB auf den Fundus F und für den Patienten scharf sichtbar abgebildet werden kann.

Vorteilhaft ist ein zum Flächenmittelpunkt FMP radial ausgerichteter Steg ST vorhanden, der als mechanische Halterung der Makulablende MB dient und der mit Wechsel des zu untersuchenden Auges A vom rechten zum linken Auge A um 180° gegen die Leuchtfeldmitte gedreht werden kann.

Als eine vorteilhafte Ausführungsvariante kann der Steg ST derart breit und transparent ausgeführt werden, dass er die Papille P abdeckt, um die im Bereich der Papille P reflektierte Intensität des Messlichtes an die der umgebenden Netzhaut und damit an den Dynamikbereich des digitalen Bildsensors 2 anzupassen.

In einer vorteilhaften Ausbildung des Ausführungsbeispieles für weitere medizinische Fragestellungen sind Mittel vorgesehen, die es erlauben, die Makulablende MB radial im Leuchtfeld LF zu verschieben. Dadurch, dass das zu untersuchende Auge A durch Verfolgung der Fixationsmarke FM der Makulablende MB so folgt, dass stets die Makula M durch die Makulablende MB abgedeckt wird, werden die Papille P und einzelne ausgewählte Netzhautgefäßbäume im Leuchtfeld LF für Messungen zentraler angeordnet und können untersucht werden.

Eine andere vorteilhafte Ausführung ist die feste Anordnung einer oder mehrerer Makulablenden MB am inneren Rand der Feldblende FB. Der Vorteil einer solchen Ausführung ist die schnelle, einfache, für verschiedene Augen standardisierte und für Wiederholungsmessungen reproduzierbare Einstellung des Fundus F bzw. der Messstellen.

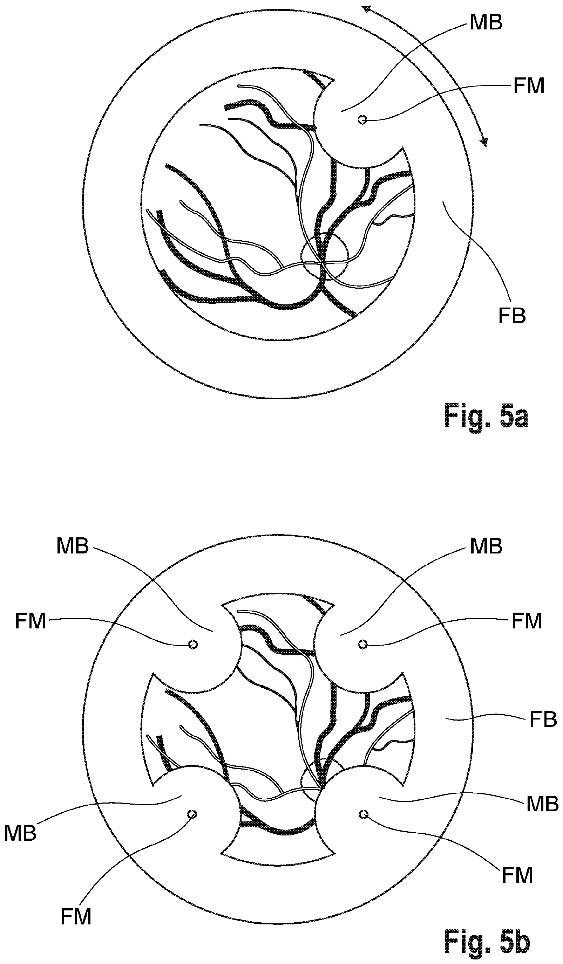

In **Fig. 5a** ist eine Ausführung gezeigt, in der eine Makulablende MB an der Feldblende FB fest verbunden oder monolithisch ausgeführt angeordnet ist. Durch eine Drehung der Feldblende FB um ihren Mittelpunkt, der auf der optischen Achse des Beleuchtungsstrahlenganges 1.1 liegt, wird das Auge A kreisend maximal ausgelenkt, womit nacheinander in Summe ein größtmöglicher Bereich des Fundus F ausgeleuchtet, abgebildet und folglich untersucht werden kann.

In **Fig. 5b** ist eine Ausführung gezeigt, in der vier Makulablenden MB jeweils paarweise gegenüberliegend bzw. jeweils um 90° zueinander versetzt am inneren Rand der Feldblende FB ausgebildet sind. Für diesen Fall ist es angedacht, dass der Patient mit dem Auge A nacheinander wenigstens die Fixationsmarken FM der zwei sich gegenüberliegenden Makulablenden MB fixiert, womit vorbestimmte Bereiche des Fundus F beleuchtet und abgebildet werden.

Die Daten- und Bildverarbeitungseinheit 5 ist vorzugsweise so auslegt, das die Abbildung der Makulablende MB, gegebenenfalls mit Steg ST, im Bild, das dem Untersucher angeboten wird, softwareseitig elektronisch sauber ausgeblendet wird.

Die Feldblende FB, die ein Leuchtfeld LF am Fundus F begrenzt, wird bevorzugt mit einem kleineren Querschnitt auf der Empfangsfläche 2.1 des digitalen Bildsensors 2 abgebildet, als es der Querschnitt der Empfangsfläche 2.1 ist. Dadurch entsteht im Unterscheid zum Stand der Technik, in dem die Größe des Bildes der Feldblende FB regelmäßig an die Größe der Empfangsfläche 2.1 des digitalen Bildsensors 2 angepasst wird und somit die Empfangsfläche 2.1 voll ausgeleuchtet wird, ein Differenzbereich 2.2 außerhalb des Bildes des Fundus F, der zur Berechnung der Streulichtverteilung sowie zur Kontrolle einer reflexfreien und streulichtarmen Einstellung der Funduskamera 1 auf das Auge A genutzt werden kann. Dabei ist es unerheblich, ob Teile des Differenzbereiches 2.2 dem Untersucher als Einstellhilfe zugänglich gemacht werden oder für automatische Einstellverfahren genutzt werden. Die Daten- und Bildverarbeitungseinheit 5 bestimmt die Helligkeitsverteilung auf der Empfangsfläche 2.1 des digitalen Bildsensors 2 innerhalb der Abbildung der Makulablende MB und im Differenzbereich 2.2 und errechnet damit über einen Approximationsalgorithmus die Streulichtverteilung im Bild des Fundus F auf dem digitalen Bildsensor 2 und korrigiert entsprechend das Bild.

Der Beleuchtungsstrahlengang 1.1 enthält weiterhin als Beleuchtungseinheit 3 erfindungsgemäß eine vorzugsweise adaptiv strukturierbare kreis- oder ringförmige Anordnung von kleinen Lichtquellen z. B. drei Gruppen von LEDs mit unterschiedlichen spektralen Eigenschaften, vorzugsweise im blauen, grünen und roten Spektralbereich, die im Beleuchtungsstrahlengang 1.1 in einer konjugierten Ebene AP" zur Ebene der Augenpupille AP angeordnet sind. Über eine differenzierte Ansteuerung der LEDs wird durch die jeweils angesteuerten (aktiven) LEDs eine adaptive Beleuchtungsstruktur gebildet.

Diese LEDs werden über die adaptive Steuereinheit 4 so angesteuert, dass die LED-Lichtintensität der farblich unterschiedlichen LEDs getrennt und unabhängig voneinander modelliert wird. Die Modulation des LED-Lichtes soll sowohl die Einstellung der Intensität von kontinuierlichem Licht als Messlicht ermöglichen, als auch die Einstellung von Stimulationslicht als Wechsel zwischen hoher und geringer Intensität, mit einstellbaren Parametern der Frequenz, des Modulationsgrades und der Wechsellichtform (z. B. wellenförmiger bis sprungförmiger, symmetrischer oder asymmetrischer Wechsel zwischen Hell- und Dunkelphase). Auch kann die Beleuchtungsstruktur, die jeweils durch die zeitlich angesteuerten (aktiven) LEDs einer Gruppe, bzw. eines Spektralbereiches bestimmt wird, an die Anforderungen für verschiedene Untersuchung angepasst werden. Z.B. kann die Beleuchtungsstruktur durch die zeitlich und örtlich angesteuerten aktiven LEDs als zeitlich wechselnde schmalere oder breitere Ringe, Halbringe, Ringsegmente oder Punkte gebildet werden, was zur Streulicht- oder Reflexionslichtverminderung und zur Anpassung an die Öffnung der Augenpupille AP eingesetzt werden kann.

Die Öffnung der Augenpupille kann sich je nach Untersuchung im Nonmyd-Modus oder Mydriatischen Modus, den Lichtverhältnissen und vom Patienten abhängig individuell stark unterscheiden. Um die Lichtbelastung des Patienten und optimale Abbildungsverhältnisse für die Bildgebung und Messung zu schaffen wird ein Außendurchmesser dₐ der Beleuchtungsstruktur an die Öffnung der Augenpupille AP angepasst. Ein Innendurchmesser dᵢ der Beleuchtungsstruktur, wird an die Größe der Abbildung AB' einer im Beobachtungsstrahlengang 1.2 in einer zur Augenpupille AP konjugierte Ebene AP" stehenden Aperturblende AB angepasst. Vorteilhaft ist der Innendurchmesser dᵢ der Beleuchtungsstruktur größer als der Durchmesser der Abbildung AB' der Aperturblende AB. Durch die Anpassung dieses strahlenfreien Raumes zwischen Beleuchtungs- und Abbildungslicht kann der Einfluss von Reflektionen oder Streulicht in Abhängigkeit von den individuellen Gegebenheiten des Patientenauges reduziert werden.

Auch kann eine während der Untersuchung rotierende Strukturänderung bzw. differenziert lokale Ansteuerung der LEDs zur Erfassung von Bildfolgen mit unterschiedlichen Beleuchtungsstrukturen eingesetzt werden, womit der Winkel, unter dem das auf ein Fundusobjekt (z.B. Gefäße ) einfallenden Beleuchtungsstrahlen so variiert werden kann, das Gefäßreflexionen reduziert und lokale Bildkontraste erhöht werden.

Die adaptive Anpassung, insbesondere des Außendurchmessers dₐ der Beleuchtungsstruktur, ermöglicht auch, die dynamische Gefäßanalyse, im mydiadrischen Modus, sehr schnell in den Modus der nonmydriatischen statischen Gefäßanalyse und umgekehrt umzuschalten. Gleichzeitig kann über diese Adaptivität die Scharfstellung des Fundus F über das Prinzip der Scheiner'schen Blenden erfolgen.

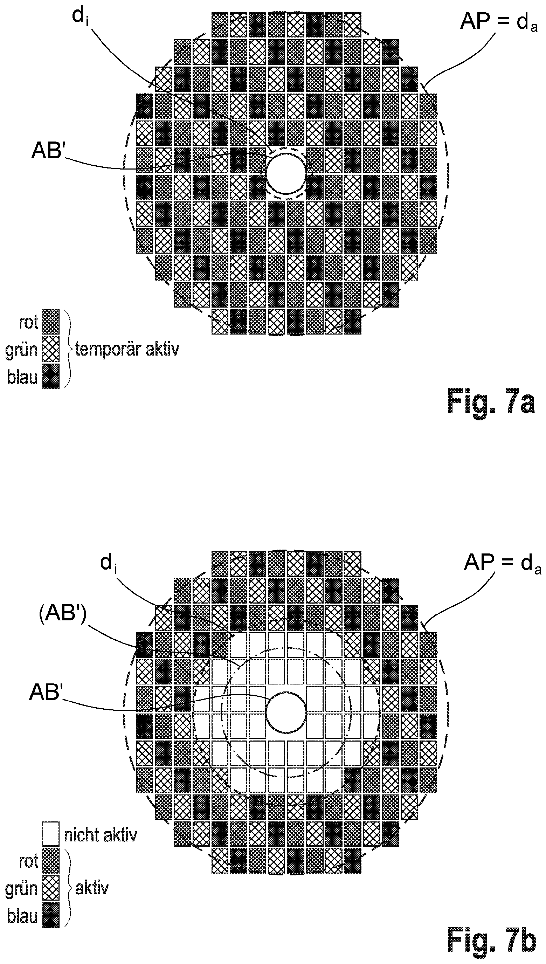

In den **Fig. 7a** bis **7e** sind Beispiele für verschiedene Ausführung der Beleuchtungseinheit 3 als adaptive, strukturierbare Anordnung von Lichtquellen gezeigt.

Dargestellt ist jeweils eine Abbildung der Beleuchtungseinheit 3 in der Ebene der Augenpupille AP zusammen mit einer Abbildung AB', der im Beobachtungsstrahlengang 1.2 stehenden Aperturblende AB. Die Beleuchtungseinheit 3 stellt vorteilhaft eine kreis- oder ringförmige Anordnung von LEDs in einer Ringform dar.

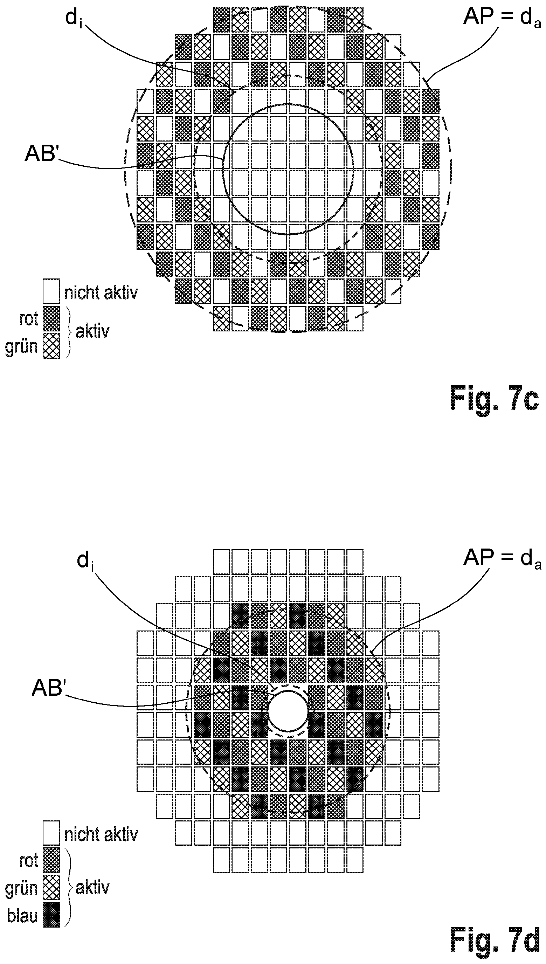

In den **Fig. 7b** und **7d** sind vergleichsweise eine Augenpupille AP mit großer und kleiner Öffnung dargestellt. Die Beleuchtungsstruktur, gebildet durch aktiv geschaltete LEDs; durch verschiedene Schraffuren dargestellt, und die Aperturblende AB, die z.B. als eine Irisblende ausgeführt in ihrem Durchmesser verstellbar ist, sind so angepasst, dass ein optimaler Lichtstrom in das Auge A gelangt oder ein optimaler Durchmesser der Aperturblende AB für eine hohe Bildauflösung eingestellt ist. Auch kann bei Änderung des Außendurchmessers dₐ, der Beleuchtungsstruktur und entsprechender Anpassung des Innendurchmessers dᵢ deren Fläche konstant gehalten werden, um den einfallenden Lichtstrom durch Kompensation stabil zu halten. Dabei ist der Außendurchmesser dₐ optimal an die Öffnung der Augenpupille AP bzw. der Augeniris angepasst.

In den **Fig. 7a** und **7b** ist vergleichsweise, bei einer gleichen Öffnung der Augenpupille AP dargestellt, dass der Innendurchmesser dᵢ weit geöffnet wird. Damit entsteht entweder ein großer strahlfreier Raum zwischen dem Beleuchtungsstrahlenbündel, nach innen begrenzt durch den Innendurchmesser dᵢ, und dem Beobachtungsstrahlenbündel, begrenzt durch die Abbildung AB' (als Volllinie in Fig. 7b) der kleinen Aperturblende AB, womit eine Reduzierung von Streulicht insbesondere bei älteren Patienten erreicht wird. Oder bei einer großen Aperturblende AB (AB'als Strich-Punkt-Linie in Fig. 7b) werden vergleichsweise die Abbildungseigenschaften verbessert und dem digitalen Bildsensor 2 mehr Licht zur Verfügung gestellt.

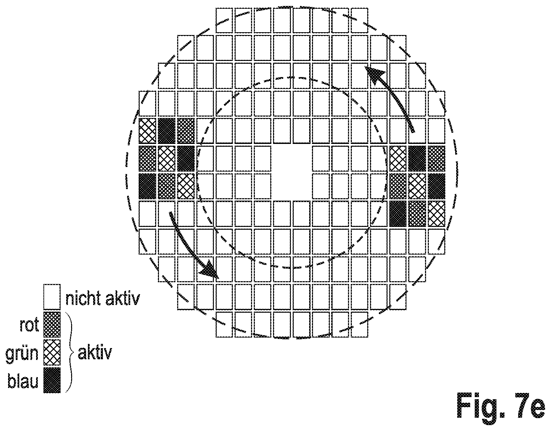

Bei herkömmlichen Beleuchtungseinheiten kommt es häufig zu dem Problem, dass der Beleuchtungsstrahlengang durch hängende Lieder, lange Wimpern oder auch eine schlitzförmige Augenform abgeschattet wird, wodurch weniger Licht in das Auge A gelangt und es zur Verschlechterung der Bildqualität kommt, was bis zum Abbruch der Untersuchung führen kann. Durch ein Zu- und Abschalten von LEDs kann wie in **Fig. 7e** dargestellt, eine Beleuchtungsstruktur geschaffen werden die Abschattungen ausweicht. Auch gibt es bei älteren Patienten häufig das Problem, das Linsentrübungen die Bildqualität erheblich beeinträchtigen. Auch in diesem Fall kann durch eine Änderung der Lage der Beleuchtungsstruktur, wie beispielhaft in **Fig. 7d** gezeigt, der Beleuchtungsstrahlengang an stärkeren Trübungsgebieten der Augenlinse vorbeigeführt werden. Hierzu kann z.B., durch eine kreisende Ansteuerung einer solchen Beleuchtungsstruktur der Beleuchtungsstrahlengang in seiner Lage optimiert werden.

Auch zur Beseitigung von störenden Gefäßreflexen auf dem Augenhintergrund oder zur kontrastreicheren Darstellung von Gefäßen und anderen Objekten am Augenhintergrund kann vorteilhaft über eine differenzierte Ansteuerung der LEDs die Einstrahlrichtung von Beleuchtungsstrahlenbündeln auf den Augenhintergrund verändert werden.

In **Fig. 7c** ist beispielhaft gezeigt, dass nur die grünen und roten LEDs aktiviert sind, die im Ausführungsbeispiel das Messlicht erzeugen.

Eine andere vorteilhafte Ausführungsvariante für einen digitalen Bildsensor 2 ist die Aufnahme von Farbbildern durch einen monochromatischen Bildsensor. Dazu werden in schneller Folge jeweils drei monochromatische Bilder zusammengefasst, wobei jedem der drei monochromatischen Bilder eine andere LED-Farbe als Beleuchtung zugeordnet wird. Die drei Bilder werden anschließend jeweils zu einem Farbbild (Bild mit drei zugeordneten Farbkanälen) zusammengefasst.

Die adaptive Steuereinheit 4 ist mit einer Daten- und Bildverarbeitungseinheit 5 verbunden, die wiederum mit dem digitalen Bildsensor 2 verbunden ist. Die Frequenz des Flickerlichtes (Wechsel zwischen hell und dunkel) wird durch ein Synchronisationssignal gesteuert, das in diesem Ausführungsbeispiel von dem digitalen Bildsensor 2 erzeugt und an die Steuereinheit 4 übergeben wird, und zur Synchronisation sämtlicher während der Verfahrenschritte gebildeter Signale mit der durch den digitalen Bildsensor 2 aufgenommenen Bildfolge synchronisiert. Für die Erfindung ist es dabei egal, ob das Synchronisationssignal von dem digitalen Bildsensor 2 oder durch die Daten- und Bildverarbeitungseinheit 5 vorgegeben wird und die Aufnahme der Bilder der Bildfolge steuert.

Der digitale Bildsensor 2 nimmt mit einer Bildfolgefrequenz von vorzugsweise 25 Hz Bilder vom Fundus F auf, womit sich vorzugsweise 12,5 Hz als Flickerfrequenz ergibt. Erfindungsgemäß kann aber auch jede andere Bildfolgefrequenz synchronisiert zu einer Flickerfrequenz für die Vorrichtung und das Verfahren eingesetzt werden. Dabei kann für verschiedene Fragestellungen auch eine variable Bildfolge- und Flickerfrequenz zum Einsatz kommen.

Die Daten- und Bildverarbeitungseinheit 5 selektiert die Papille P, die Abbildung der Makulablende MB gegebenenfalls mit Steg ST sowie arterielle und venöse große Gefäße der Netzhaut in jedem Bild zeichnet die Bewegungskoordinaten des Fundus F der Fixationsmarke FM folgend auf und benutzt diese zur Bewegungskorrektur der Bilder der Bildfolge bzw. der Messdaten und -signale.

Ferner ist eine Einheit zur Erzeugung von Durchmessersignalen 6 vorgesehen, die die Gefäßdurchmesser an den selektierten Gefäßsegmenten bestimmt und zeit- und ortsabhängig gefäßsegmentweise Durchmessersignale D(t,x,y) für eine Bildfolge erzeugt und diese Signale an die Signalanalyseeinheit 7 übergibt. Dort werden aus den Signalen der Gefäßsegmente durch Zusammenfassung mehrerer Gefäßsegmente gemittelte Durchmessersignale D(t,x,y) für ganze Gefäßabschnitte durch Mittelwertbildung gebildet und dem Untersucher zur Präsentation grafisch dargestellt und ausgegeben. In der Signalanalyseeinheit 7 werden auch typische, die Endothelfunktion beschreibende Parameter der Gefäße wie z. B. das Flickerdilatationsmaximum FDₘₐₓ in der Stimulationsphase SP berechnet und über die Ergebnis- und Präsentationseinheit 8 und die Ein- und Ausgabeeinheit 9 ausgegeben. Die Ergebnis- und Präsentationseinheit 8 dient zudem zur Erstellung von Mappingbildern.

Nachfolgend wird das erfindungsgemäße Verfahren anhand eines Ausführungsbeispiels beschrieben.

### Schritt 0:

Dem Untersucher wird ein Untersuchungsprogramm-Menü zu verschiedenen Untersuchungen mit unterschiedlichen medizinischen Fragestellungen angeboten. Eingestellt werden mit der Wahl der Untersuchungsparameter die Anordnung der wenigstens einen Makulablende MB, die Parameter des Fixationslichtes, sofern die Fixationsmarke FM durch eine selbst leuchtende Marke gebildet wird, die Parameter des Messlichtes und die Parameter des Flickerlichtes.

Der Untersucher kann zwischen dem Einstellen
0-1: frei gewählter Parameter (freie Parameterwahl),
0-2: von Vergleichsparametern (Vergleichsmodus) und
0-3: von Wiederholparametern (Wiederholmodus)
wählen, wie in den nachfolgenden Verfahrensschritten beschrieben.

### Schritt 0-1: Freie Parameterwahl

Für Fragestellungen der Forschung ist eine freie Parameterwahl häufig sinnvoll. Dem Untersucher werden vorzugsweise die nachfolgenden Parameter zur automatischen Voreinstellung angeboten und nach der Auswahl wird der Parametersatz unter einem vom Untersucher zu vergebenden Namen als neues Programm für Vergleichs- und Wiederholungsuntersuchungen gespeichert.

### Schritt 0-1-1: Einstellung von Art und Lage der Makulablende MB sowie des Fixationspunktes (Fixationsparameter)

Die Art und die Lage der einen oder mehreren Makulablenden MB und gegebenenfalls des Stegs ST werden zur manuellen Einstellung als Bild dem Untersucher angezeigt oder automatisch voreingestellt.

Der Untersucher wählt dann die Fixationsart aus, indem er folgende Fixationsparameter für die Fixationsmarke FM einstellt:
- spektrale Festlegung der geometrischen Fixationsstruktur (Kreuz, Punkt, Ring...)
- Festlegung der Fixationsfarbe
- Festlegung der Fixationsintensität
- Festlegung der zeitlichen Fixationsintensitätsänderungen (z. B. Blinkfrequenz)
- oder Nutzung einer Öffnung in der Makulablende MB als Fixationsmarke FM, die durch das Flicker- und Messlicht ausgeleuchtet wird

Schritt 0-1-2: Einstellung des Messlichtes (Messlichtparameter)
- Festlegung des Spektralbereiches (vorzugsweise grün), für spezielle Fragestellungen kann auch das Messlicht aus verschiedenen Spektralbereichen zum Einsatz kommen
- Festlegung der Intensität (manuell oder automatisch nachregelbar, gesteuert durch Bildhelligkeit)
- Festlegung des Zeitverhaltens während der Stimulationsphase SP

So können das Flickerlicht zur Stimulation und das Messlicht unabhängig voneinander für die medizinische Fragestellung aufeinander abgestimmt werden.

Schritt 0-1-3: Einstellung der Flickerlichtparameter
- Einstellung auf Luminanzflicker oder Farbflicker

Beim Luminanzflicker wird der festgelegte Spektralbereich des Flickerlichtes nur entsprechend der anderen Flickerparameter moduliert. Im Fall des Farbflickers wechselt das Flickerlicht nur den Spektralbereich mit der Flickerfrequenz, was ein wechselseitiges Umschalten der farblich unterschiedlichen LEDs bedeutet.
- die Einstellung der Spektralbereiche der farblichen LEDs wird je nach Flickerart vorgenommen, z. B. bei Farbflicker wird der Wechsel des Flickerlichtes von einer blauen LED mit einer grünen LED festgelegt
- Einstellung der Modulation des Flickerlichtes (Modulationsparameter)

Im vorliegenden Beispiel kann der Untersucher die Stimulationsform für jede Halbperiode des Flickerlichtes mit folgenden Parametern festlegen:
- Intensitätsmaximum
- Intensitätsminimum
- Modulationstiefe
- Intensitätsanstieg
- Intensitätsabfall
- Länge des Intensitätsmaximums
- wellenförmige oder sprungförmige Modulation

Schritt 0-1-4: Einstellung der Untersuchungsphasen (Phasenparameter)
- eingestellt werden die Länge der Untersuchungsphasen: Baseline-Phase BP, Stimulationsphase SP und Nachphase NP
- im Fall einer adaptiven Stimulationsphase SP werden eingestellt:
   - eine Mindestdauer der Stimulationsphase SP
   - eine maximale Dauer der Stimulationsphase SP
   - und die Abbruchparameter für die Stimulationsdauer

### Schritt-0-1-5:

Alle frei gewählten Parameter werden in einem Parametersatz zusammengefasst und mit einem Spezial-Untersuchungsnamen gespeichert und bei erneuter Wahl des Untersuchungsmenüs angeboten.

### Schritt 0-2: Vergleichsmodus (sichert gleiche Untersuchungsbedingungen für verschiedene Augen A für die gleiche medizinische Fragestellung)

Aus dem Untersuchungsmenü wird das gewünschte Untersuchungsprogramm für die medizinische Fragestellung herausgesucht und der zugehörige Parametersatz zu dem ausgewählten Untersuchungsprogramm wird geladen. Über vorgesehene Steueralgorithmen werden die LEDs der Vorrichtung entsprechend angesteuert, womit das Messlicht und das Flickerlicht variabel und adaptiv an das ausgewählte Untersuchungsprogramm angepasst werden.

### Schritt 0-3: Wiedermodus (sichert gleiche Untersuchungsbedingungen in Folgesitzungen für das gleiche Auge A) mit Referenzmessorten

Über die patientenbezogene Datenbank wird das schon einmal untersuchte Auge A herausgesucht und die von der letzten Untersuchung gespeicherten Datensätze zu den selektierten Gefäßen und der Parametersatz der durchgeführten Untersuchung voreingestellt.

Während der Einstellung der Vorrichtung auf das Auge A sichert die Bewegungskorrektur die exakte Übereinstimmung der erfassten Bereiche des Fundus F zwischen den Sitzungen.

Nach Einstellung aller Parameter beginnt das Untersuchungsverfahren.

### Schritt 1:

Der Kopf des Patienten wird über eine Kopf- und Kinnstütze fixiert. Der Patient wird aufgefordert, auf die Fixationsmarke FM auf der dunklen Makulablende MB zu schauen.

### Schritt 2:

Die Vorrichtung wird derart auf das zu untersuchende Auge A mittels eines Kreuztisches eingestellt, dass sich ein streulichtarmes und reflexfreies Bild des Fundus F ergibt.

### Schritt 3:

Das Bild des Fundus F und die Makulablende MB werden scharf gestellt, und durch Verdrehung des Stegs ST wird der Steg ST der Makulabelnde MB so gestellt, dass er die Papille P des zu untersuchenden Auges A abdeckt.

### Schritt 4:

Der Messvorgang der Untersuchung wird mit Beginn der Aufnahme einer Bildfolge während der Baseline-Phase BP gestartet, bei der nur Messlicht und kein Flickerlicht zum Einsatz kommt.

### Schritt 5:

### Durch Algorithmen:

- 5.1: wird die Lage des Stegs ST im Leuchtfeld LF erkannt und damit das untersuchte Auge A (rechts oder links) automatisch identifiziert.
- 5.2: werden die zentralen Restreflexe der Ophthalmoskoplinse OL mittig erkannt und elektronisch ausgeblendet.
- 5.3: wird die Abbildung der ersten Feldblende FB des Beleuchtungsstrahlenganges 1.1 der Funduskamera 1 auf dem Fundus F identifiziert.
- 5.4: wird außerhalb des Leuchtfeldes LF und im nicht durch die Makulablende MB ausgeblendeten Bereich die Streulichtverteilung berechnet und vom aufgenommenen Bild des Fundus F abgezogen.
- 5.5: werden die Bilder bezüglich der Augenbewegungen bewegungskorrigiert.
- 5.6: wird die Papille P selektiert und ausgeblendet, z. B. für Anwendungen für die statische Gefäßanalyse.
- 5.7: werden die großen arteriellen und venösen Gefäße der nichtabgedeckten Netzhaut selektiert und gespeichert.

### Schritt 6:

Durch Algorithmen zur Durchmessermessung werden Gefäßdurchmesser entlang der selektierten Gefäße Gefäßsegment für Gefäßsegment ermittelt, ortskorrigiert gespeichert und dem Synchronisationssignal und damit den einzelnen Bildern der Bildfolge zugeordnet. Aus diesen Daten werden Durchmessersignale D(t,x,y) für jedes Gefäßsegment gebildet.

### Schritt 7:

Zunächst wird die Baseline-Phase BP mit einer Baseline-Zeit gestartet. Danach schließt sich automatisch die Stimulationsphase SP mit der Stimulationszeit (Flickerperiode) und dem für die Flickerlichtstimulation übergebenden Parametersatz an.

### Schritt 8:

Während der Stimulationsphase SP werden alle Gefäßsignale auf ihren jeweils mittleren Baseline-Wert (aus der Baseline-Phase BP ermittelt) prozentual normiert. Die flickerbedingten Änderungen aller Gefäßsignale während der Stimulationsphase SP werden getrennt für Arterien Av und Venen Vv gemittelt und bezüglich ihrer Streuung und Dilatation bewertet. Bei Wahl der adaptiven Stimulationsphase SP wird erfindungsgemäß die Stimulationszeit vom Untersuchungsergebnis abhängig gemacht. Unterschreitet der Anstieg der Flickerdilatation und die Streuung der mittleren Flickerdilatation einen vorgegebenen Schwellwert nach 20 s, wird die Stimulationsphase SP beendet.

Die mittlere Flickerdilatation aller arteriellen und venösen Gefäßsegmente wird getrennt voneinander ausgegeben.

### Schritt 9:

Nach Beendigung der Stimulationsphase SP beginnt die Nachphase NP der Untersuchung, das Flickerlicht wird abgeschaltet und die kontinuierlichen Messungen werden fortgesetzt, bis die Nachphase NP nach der voreingestellten Zeit beendet wird. Auch die Nachphase NP kann adaptiv gestaltet werden, indem sie beendet wird, wenn die mittleren Signaländerungen und die Streuung der Signalwerte einen Schwellenwert unterschreiten oder ein anderes Kriterium zur Beendigung eingesetzt wird. Die Stimulationsphase SP und die Nachphase NP können zur Mittelwertbildung der Signale mehrfach, bevorzugt dreimal im Wechsel wiederholt werden.

### Schritt 10:

Aus den Durchmessersignalen D(t,x,y) werden über alle Gefäßsegmente getrennt für Arterien Av und Venen Vv weitere mittlere Parameter wie z. B. mittlere Parameter der Vasomotorik in der Baseline oder der dem Dilatationsabfall folgenden Konstriktion gebildet.

### Schritt 11:

Es wird ein mittleres arterielles und ein mittleres venöses Gefäßsignal D=f(t) über alle Gefäßsegmente gebildet und als Untersuchungsergebnis gemeinsam mit den mittleren Parametern als Untersuchungsprotokoll ausgegeben.

### Schritt 12:

Für jeden Parameter, insbesondere für das Flickerdilatationsmaximum FDₘₐₓ der arteriellen und venösen Gefäßsegmente, werden den Werten Farben zugeordnet, die dann als Funktionsimaging im Bild vom Fundus F ortsrichtig überlagert dargestellt werden. Rote Segmente kennzeichnen ein fehlendes Flickerdilatationsmaximum FDₘₐₓ und grüne Segmente kennzeichnen ein gesundes Flickerdilatationsmaximum FDₘₐₓ.

Für das erfindungsgemäße Verfahren ist es egal, ob die Einstellung und bestimmte Verfahrensschritte der Auswertung manuell oder automatisch durchgeführt werden.

Eine vorteilhafte Ausführung der erfindungsgemäßen Vorrichtung und des erfindungsgemäßen Verfahrens ist die optoelektronische Realisierung der Makulablende MB und weiterer Blenden in der zum Fundus F konjugierten Ebene F‴ im Beleuchtungsstrahlengang 1.1, z. B. durch ein Transmissionsdisplay, dessen Pixel unabhängig voneinander elektronisch in ihrer Transmission einstellbar sind. Während des erfindungsgemäßen Verfahrens wird dann softwareseitig die Ansteuerung des Displays entsprechend der einzelnen Verfahrensschritte analog der Einstellung einer mechanischen Makulablende MB vorgenommen.

### Bezugszeichenliste

- NVK: neurovaskuläre Kopplung
- RVA: Retinal Vessel Analyzer der Firma Imedos
- DVA: Dynamic Vessel Analyzer der Firma Imedos
- D(t,x,y): Durchmessersignal als Funktion der Zeit und des Ortes x, y auf dem Fundus F
- FDₘₐₓ: Flickerdilatationsmaximum
- BP: Baseline-Phase (Aufzeichnung der Signale ohne Stimulation)
- SP: Stimulationsphase (Aufzeichnung der Signale während der Stimulation)
- NP: Nachphase (Aufzeichnung der Signale nach der Stimulation)

- 1: Funduskamera
- 1.1: Beleuchtungsstrahlengang
- 1.2: Beobachtungsstrahlengang
- 2: digitaler Bildsensor
- 2.1: Empfangsfläche
- 2.2: Differenzbereich
- 3: Beleuchtungseinheit
- 4: Steuereinheit
- 5: Daten- und Bildverarbeitungseinheit
- 6: Einheit zur Erzeugung von Durchmessersignalen
- 7: Signalanalyseeinheit
- 8: Ergebnis- und Präsentationseinheit
- 9: Ein- und Ausgabeeinheit

- A: Auge
- F: Fundus
- M: Makula
- P: Papille
- V: Foveola
- Vv: Vene
- Av: Arterie
- AP: Augenpupille
- MB: Makulablende
- FMP: Flächenmittelpunkt der Makulablende
- FM: Fixationsmarke
- ST: Steg
- FB: Feldblende
- KL: Kollimatorlinse
- FL: Feldlinse
- OL: Ophthalmoskoplinse
- CO: Objektivlinse
- LB: Lochblende
- AB: Aperturblende im Beobachtungsstrahlengang 1.2
- AB': Bild der Aperturblende AB in der Augenpupille AP
- F‴: konjugierte Ebene zum Fundus F im Beleuchtungsstrahlengang 1.1
- F', F": konjugierte Ebenen zum Fundus F im Beobachtungsstrahlengang 1.2
- AP": konjugierte Ebene zur Augenpupille AP im Beleuchtungsstrahlengang 1.1
- AP': konjugierte Ebene zur Augenpupille AP im Beobachtungssstrahlengang 1.2
- LF: Leuchtfeld
- dᵢ: Innendurchmesser eine Beleuchtungsstruktur
- dₐ: Außendurchmesser einer Beleuchtungsstruktur

## Patentansprüche

1. Vorrichtung zur Untersuchung der retinalen vaskulären Endothelfunktion der Gefäße der Netzhaut am Fundus (F) eines Auges (A), enthaltend eine Funduskamera (1) mit einem Beobachtungsstrahlengang (1.2) mit einem digitalen Bildsensor (2) zur Aufnahme von Bildfolgen von Bildern von Bereichen des Fundus (F), auf den der Beobachtungsstrahlengang (1.2) der Funduskamera (1) scharf eingestellt ist, und mit einem Beleuchtungsstrahlengang (1.1), in dem in einer zu der Augenpupille (AP) des Auges (A) konjugierten Ebene (AP") eine Beleuchtungseinheit (3) angeordnet ist, zur Beleuchtung des Fundus (F) mit einem Mess- und einem Flickerlicht innerhalb eines Leuchtfeldes (LF) auf dem Fundus (F), das durch die Abbildung einer Feldblende (FB) begrenzt ist, die in einer zum Fundus (F) konjugierten Ebene (F‴) im Beleuchtungsstrahlengang (1.1) steht, wobei in der zum Fundus (F) konjugierten Ebene (F‴) im Beleuchtungsstrahlengang (1.1) wenigstens eine Makulablende (MB) angeordnet ist, an der sich jeweils eine Fixationsmarke (FM) befindet, so dass eine der wenigstens einen Makulablende (MB) die Makula (M) auf dem Fundus (F) abdeckt, wenn das Auge (A) auf die Fixationsmarke (FM) der einen Makulablende (MB) fixiert.

2. Vorrichtung nach Anspruch 1, **dadurch gekennzeichnet,**
**dass** die Makulablende (MB) eine mechanische Blende ist.

3. Vorrichtung nach Anspruch 1, **dadurch gekennzeichnet,**
**dass** die Makulablende (MB) eine optoelektronische Blende ist.

4. Vorrichtung nach Anspruch 3, **dadurch gekennzeichnet,**
**dass** die Makulablende (MB) ein Transmissionsdisplay ist.

5. Vorrichtung nach Anspruch 2, **dadurch gekennzeichnet,**
**dass** genau eine Makulablende (MB) vorhanden ist, die in der zum Fundus (F) konjugierten Ebene (F‴) im Beleuchtungsstrahlengang (1.1) angeordnet ist, und die Fixationsmarke (FM) eine punktförmige Öffnung in einem Flächenmittelpunkt (FMP) der Makulablende (MB) ist.

6. Vorrichtung nach Anspruch 5, **dadurch gekennzeichnet,**
**dass** an die Makulablende (MB) ein zu deren Flächenmittelpunkt (FMP) radial ausgerichteter teiltransparenter Steg (ST) angrenzt, mit dem die Papille (P) am Fundus (F) überdeckt werden kann, sodass die Strahlungsintensität einer Abbildung der Papille (P) auf einen Dynamikbereich des digitalen Bildsensors (2) angepasst werden kann, der für die Strahlungsintensität einer Abbildung der die Papille (P) umgebenden Bereiche des Fundus (F) ausgelegt ist.

7. Vorrichtung nach Anspruch 6, **dadurch gekennzeichnet,**
**dass** eine weitere teiltransparente Blende mit einstellbarer Transparenz zur Abdeckung der Papille (P) vorhanden ist.

8. Vorrichtung nach Anspruch 6 oder 7, **dadurch gekennzeichnet,**
**dass** die Makulablende (MB) in der zum Fundus (F) konjugierten Ebene (F‴) im Beleuchtungsstrahlengang (1.1) verschiebbar ist, wobei deren Flächenmittelpunkt (FMP) innerhalb der Feldblende (FB) liegend verbleibt, womit unterschiedliche ausgewählte Bereiche des Fundus (F) von dem Leuchtfeld (LF) ausgeleuchtet werden und Bildfolgen von Bildern der unterschiedlichen ausgewählten Bereichen des Fundus (F) aufgenommen werden können.

9. Vorrichtung nach Anspruch 1, **dadurch gekennzeichnet,**
**dass** genau eine Makulablende (MB) an der Feldblende (FB) so ausgebildet ist, dass die Fixationsmarke (FM) an einen inneren Rand angrenzend innerhalb der Feldblende (FB) liegt, und die Feldblende (FB) um eine optische Achse des Beleuchtungsstrahlenganges (1.1) drehbar ist, womit unterschiedliche ausgewählte Bereiche des Fundus (F) von dem Leuchtfeld (LF) ausgeleuchtet werden und Bildfolgen von Bildern der unterschiedlichen ausgewählten Bereiche des Fundus (F) aufgenommen werden können.

10. Vorrichtung nach Anspruch 1, **dadurch gekennzeichnet,**
**dass** genau vier Makulablenden (MB) an der Feldblende (FB) paarweise diagonal gegenüberliegend so ausgebildet sind, dass die jeweilige Fixationsmarke (FM) an einen inneren Rand angrenzend innerhalb der Feldblende (FB) liegt, womit unterschiedliche vorbestimmte Bereiche des Fundus (F) von dem Leuchtfeld (LF) ausgeleuchtet werden und Bildfolgen von Bildern der unterschiedlichen vorbestimmten Bereiche des Fundus (F) aufgenommen werden können.

11. Vorrichtung nach Anspruch 1, **dadurch gekennzeichnet,**
**dass** die Beleuchtungseinheit (3) zur Realisierung verschiedener Beleuchtungsstrukturen durch eine adaptive, strukturierbare Anordnung von Lichtquellen in der zur Augenpupille (AP) konjugierten Ebene (AP") gebildet ist, die örtlich, spektral und zeitlich beliebig und voneinander getrennt zu- und abgeschaltet und / oder in ihrer Intensität moduliert werden können.

12. Vorrichtung nach Anspruch 1, **dadurch gekennzeichnet,**
**dass** eine Abbildung der im Beleuchtungsstrahlengang (1.1) stehenden Feldblende (FB) auf dem digitalen Bildsensor (2) einen kleineren Querschnitt aufweist als eine Empfangsfläche (2.1) des digitalen Bildsensors (2), und die Helligkeitsverteilung in einem sich bildenden Differenzbereich (2.2) genutzt wird, um eine Streulichtverteilung zu ermitteln, mit der die Bilder der Bildfolgen korrigiert werden können.

13. Verfahren zur Untersuchung der retinalen vaskulären Endothelfunktion der Gefäße der Netzhaut am Fundus (F) eines Auges (A), enthaltend die Verfahrensschritte:
- Einstellung einer Funduskamera (1) auf das Auge (A),
- mittels einer Beleuchtungseinheit, Erzeugung von Messlicht zur Beleuchtung der Gefäße der Netzhaut am Fundus (F) und von Flickerlicht zur Stimulation der Gefäße der Netzhaut am Fundus (F) während einer Stimulationsphase (SP),
- mittels der Funduskamera, Erzeugung einer Bildfolge von Bildern eines Bereiches des Fundus (F) während einer Baseline-Phase (BP), wenigstens einer Stimulationsphase (SP) und wenigstens einer Nachphase (NP),
- Messung der Gefäßdurchmesser selektierter Gefäßsegmente der Gefäße der Netzhaut in den Bildern der erzeugten Bildfolge abhängig von Ort und Zeit,
- Bewegungskorrektur für die gemessenen Gefäßsegmente, bei der jedes Gefäßsegment einem Ort am Fundus (F) bewegungskorrigiert zugeordnet wird,
- Bildung von Durchmessersignalen (D(t,x,y)), die die gemessenen Gefäßdurchmesser als Funktion der Zeit und des Ortes des jeweils selektierten Gefäßsegmentes darstellen,
- Ableiten von Gefäßparametern aus den Durchmessersignalen (D(t,x,y)), die jeweils die Endothelfunktion des jeweils selektierten Gefäßsegmentes beschreiben, wobei die Beleuchtung und Stimulation der Makula (M) am Fundus (F) verhindert wird, indem wenigstens eine Makulablende (MB), an der sich eine Fixationsmarke (FM) befindet, auf dem Fundus (F) scharf abgebildet wird und das Auge (A) durch Fixierung auf die Fixationsmarke (FM) einer der wenigstens einen Makulablende (MB) so zu der einen Makulablende (MB) ausgerichtet wird, dass die Makula (M) durch eine Abbildung der einen Makulablende (MB) überdeckt ist.

14. Verfahren nach Anspruch 13, **dadurch gekennzeichnet,**
**dass** nacheinander Bildfolgen von Bildern ausgewählter unterschiedlicher Bereiche des Fundus (F) aufgenommen werden, jeweils nachdem genau eine auf den Fundus (F) abgebildete Makulablende (MB) in eine andere Lage verschoben wurde, so dass deren Abbildung auf dem Fundus (F) verschoben wurde und das Auge (A) die Fixationsmarke (FM) fixierend gefolgt ist.

15. Verfahren nach Anspruch 14, **dadurch gekennzeichnet,**
**dass** die Lageparameter der Lagen der Makulablende (MB) gespeichert werden und für Wiederholungs- und Folgemessungen wieder aufrufbar und einstellbar sind.

16. Verfahren nach Anspruch 13, **dadurch gekennzeichnet,**
**dass** alternativ Bildfolgen von vorgegebenen unterschiedlichen Bereichen des Fundus (F) aufgenommen werden, während vier Makulablenden (MB), die diagonal gegenüberliegend angeordnet sind, auf den Fundus (F) abgebildet werden und jeweils das Auge (A) nacheinander eine andere der Fixationsmarken (FM) fixiert hat.

17. Verfahren nach Anspruch 13, **dadurch gekennzeichnet,**
**dass** die Dauer der Baseline-Phase (BP), der Stimulationsphase (SP) und der Nachphase (NP) und die Parameter des Messlichtes und des Flickerlichtes unabhängig voneinander eingestellt werden und einem Patienten und einem Untersuchungsprogramm zugeordnet gespeichert werden, so dass sie für Wiederholungs- und Vergleichsuntersuchungen als Parametersatz aufgerufen und wieder eingestellt werden können.

18. Verfahren nach Anspruch 13, **dadurch gekennzeichnet,**
**dass** als einer der abgeleiteten Gefäßparameter das Flickerdilatationsmaximum (FDₘₐₓ) ermittelt wird und in einem Messprotokoll gemeinsam mit einer grafischen Darstellung von gemittelten Durchmessersignalen (D(t,x,y)) ausgegeben wird, die jeweils für die selektierten arteriellen und für die selektierten venösen Gefäßsegmente aus den dafür gebildeten Durchmessersignalen (D(t,x,y)) gebildet werden.

19. Verfahren nach Anspruch 18, **dadurch gekennzeichnet,**
**dass** das Flickerdilatationsmaximum (FDₘₐₓ) in einem Mappingbild als Funktionsimaging der Endothelfunktion dem jeweils zugehörigen Gefäßsegment farblich kodiert zugeordnet wird, wobei eine fehlende Gefäßdilatation oder eine Gefäßdilatation unterhalb eines vorgegebenen Schwellwertes mit rot und eine Gefäßdilatation oberhalb eines Schwellwertes, die einer gesunden Gefäßfunktion entspricht, mit grün auf dem zugehörigen Gefäßsegment markiert wird und das Mappingbild als grafisches Untersuchungsergebnis ausgegeben wird.

## Claims

1. A device for examining the retinal vascular endothelial function of the vessels of the retina at the fundus (F) of an eye (A), containing a fundus camera (1) with an observation beam path (1.2) with a digital image sensor (2) for recording image sequences of images of regions of the fundus (F) on which the observation beam path (1.2) of the fundus camera (1) is focused, and with an illumination beam path (1.1), in which an illumination unit (3) is arranged in a plane (AP") conjugate to the eye pupil (AP) of the eye (A), for illuminating the fundus (F) with a measuring light and a flicker light within a luminous field (LF) on the fundus (F), which is delimited by the mapping of a field stop (FB), which is located in a plane (F‴) conjugate to the fundus (F) in the illumination beam path (1.1), wherein
at least one macula stop (MB) is arranged in the plane (F‴) conjugate to the fundus (F) in the illumination beam path (1.1), on each of which there is a fixation mark (FM), so that at least one of the macula stops (MB) covers the macula (M) on the fundus (F) when the eye (A) fixates on the fixation mark (FM) of the one macula stop (MB).

2. The device according to claim 1, wherein the macula stop (MB) is a mechanical stop.

3. The device according to claim 1, wherein the macula stop (MB) is an optoelectronic stop.

4. The device according to claim 3, wherein the macula stop (MB) is a transmission display.

5. The device according to claim 2, wherein exactly one macula stop (MB) is present, which is arranged in the plane (F‴) conjugate to the fundus (F) in the illumination beam path (1.1), and the fixation mark (FM) is a point-shaped opening in a surface center (FMP) of the macula stop (MB).

6. The device according to claim 5, wherein the macula stop (MB) is adjoined by a partially transparent bar (ST) which is radially aligned with the center of its surface (FMP) and with which the papilla (P) on the fundus (F) can be covered, so that the radiation intensity of an image of the papilla (P) can be adapted to a dynamic range of the digital image sensor (2) which is designed for the radiation intensity of an image of the regions of the fundus (F) surrounding the papilla (P).

7. The device according to claim 6, wherein a further partially transparent stop with adjustable transparency is available to cover the papilla (P).

8. The device according to claim 6 or 7, wherein the macula stop (MB) is displaceable in the plane (F‴) conjugate to the fundus (F) in the illumination beam path (1.1), the center of its surface (FMP) remaining within the field stop (FB), whereby different selected regions of the fundus (F) are illuminated by the luminous field (LF) and image sequences of images of the different selected regions of the fundus (F) can be recorded.

9. The device according to claim 1, wherein precisely one macula stop (MB) is formed on the field stop (FB) such that the fixation mark (FM) lies adjacent to an inner edge within the field stop (FB), and the field stop (FB) is rotatable about an optical axis of the illumination beam path (1.1), whereby different selected regions of the fundus (F) are illuminated by the luminous field (LF) and image sequences of images of the different selected regions of the fundus (F) can be recorded.

10. The device according to claim 1, wherein exactly four macula stops (MB) are formed diagonally opposite one another in pairs on the field stop (FB) in such a way that the respective fixation mark (FM) lies adjacent to an inner edge within the field stop (FB), whereby different predetermined regions of the fundus (F) are illuminated by the luminous field (LF) and image sequences of images of the different predetermined regions of the fundus (F) can be recorded.

11. The device according to claim 1, wherein the illumination unit (3) for realizing various illumination structures is formed by an adaptive, structurable arrangement of light sources in the plane (AP") conjugate to the eye pupil (AP), which can be switched on and off and/or modulated in intensity locally, spectrally and temporally as desired and separately from one another.

12. The device according to claim 1, wherein an mapping of the field stop (FB) located in the illumination beam path (1.1) on the digital image sensor (2) has a smaller cross-section than a receiving area (2.1) of the digital image sensor (2), and the brightness distribution in a difference area (2.2) which is formed is used to determine a stray light distribution with which the images of the image sequences can be corrected.

13. A method for examining the retinal vascular endothelial function of the vessels of the retina at the fundus (F) of an eye (A), comprising the method steps:
- Adjustment of a fundus camera (1) to the eye (A),
- by means of an illumination unit, generation of measuring light for illuminating the vessels of the retina at the fundus (F) and of flicker light for stimulating the vessels of the retina at the fundus (F) during a stimulation phase (SP),
- by means of the fundus camera, generating an image sequence of images of a region of the fundus (F) during a baseline phase (BP), at least one stimulation phase (SP) and at least one post-phase (NP),
- Measurement of the vessel diameters of selected vessel segments of the retinal vessels in the images of the generated image sequence depending on location and time,
- Motion correction for the measured vessel segments, in which each vessel segment is assigned to a location on the fundus (F) corrected for motion,
- formation of diameter signals (D(t,x,y)), which represent the measured vessel diameters as a function of time and the location of the selected vessel segment,
- derivation of vessel parameters from the diameter signals (D(t,x,y)), each of which describes the endothelial function of the selected vessel segment, where the illumination and stimulation of the macula (M) at the fundus (F) is prevented by at least one macula stop (MB), at which a fixation mark (FM) is located, being sharply imaged on the fundus (F) and the eye (A) being aligned with the one macula stop (MB) by fixation on the fixation mark (FM) in such a way that the macula (M) is covered by an image of the macula stop (MB).

14. A method according to claim 13, wherein image sequences of images of selected different areas of the fundus (F) are recorded in succession, in each case after exactly one macula stop (MB) imaged on the fundus (F) has been displaced to a different position, so that its image on the fundus (F) has been displaced and the eye (A) has followed the fixation mark (FM) in a fixating manner.

15. A method according to claim 14, wherein the position parameters of the macula stop (MB) are saved and can be recalled and adjusted for repeat and subsequent measurements.

16. A method according to claim 13, wherein alternatively image sequences of predetermined different areas of the fundus (F) are recorded, while four macula stop (MB), which are arranged diagonally opposite one another, are imaged onto the fundus (F) and in each case the eye (A) has fixed a different one of the fixation marks (FM) in succession.

17. A method according to claim 13, wherein the duration of the baseline phase (BP), the stimulation phase (SP) and the post-phase (NP) and the parameters of the measuring light and the flicker light are set independently of each other and are stored assigned to a patient and an examination program so that they can be called up and set again as a parameter set for repeat and comparative examinations.

18. A method according to claim 13, wherein the flicker dilation maximum (FDₘₐₓ ) is determined as one of the derived vessel parameters and is output in a measurement protocol together with a graphical representation of averaged diameter signals (D(t,x,y)), which are formed in each case for the selected arterial and for the selected venous vessel segments from the diameter signals (D(t,x,y)) formed for this purpose.

19. A method according to claim 18, wherein the flicker dilation maximum (FDₘₐₓ) is color-coded in a mapping image as functional imaging of the endothelial function to the respective associated vessel segment, whereby a missing vessel dilation or a vessel dilation below a predetermined threshold value is marked with red and a vessel dilation above a threshold value, which corresponds to a healthy vessel function, is marked with green on the associated vessel segment and the mapping image is output as a graphical examination result.

## Revendications

1. Dispositif d'analyse de la fonction endothéliale vasculaire rétinienne des vaisseaux de la rétine au fond (F) d'un oeil (A), comprenant une caméra de fond d'oeil (1) avec un trajet de faisceau d'observation (1.2) avec un capteur d'images numérique (2) pour l'enregistrement de séquences d'images de zones du fond (F), sur lesquelles le trajet de faisceau d'observation (1.2) de la caméra de fond d'oeil (1) est réglé de manière nette, et avec un trajet de faisceau d'éclairage (1.1), dans lequel une unité d'éclairage (3) est disposée dans un plan (AP") conjugué à la pupille (AP) de l'œil (A), pour éclairer le fond (F) avec une lumière de mesure et une lumière de scintillement à l'intérieur d'un champ lumineux (LF) sur le fond (F), qui est délimité par l'image d'un diaphragme de champ (FB), qui se trouve dans un plan (F‴) conjugué au fond (F) dans le trajet de faisceau d'éclairage (1.1),
où
dans le plan (F") conjugué au fond (F) est disposée dans le trajet des rayons d'éclairage (1.1) au moins un diaphragme maculaire (MB), sur lequel se trouve respectivement un repère de fixation (FM), de sorte que l'un des au moins un diaphragme maculaire (MB) recouvre la macula (M) sur le fond (F) lorsque l'œil (A) se fixe sur le repère de fixation (FM) de l'un des diaphragmes maculaires (MB).

2. Dispositif selon la revendication 1, **caractérisé**
**en ce que** le diaphragme maculaire (MB) est un diaphragme mécanique.

3. Dispositif selon la revendication 1, **caractérisé**
**en ce que** le diaphragme maculaire (MB) est un diaphragme optoélectronique.

4. Dispositif selon la revendication 3, **caractérisé**
**en ce que** le diaphragme maculaire (MB) est un écran à transmission.

5. Dispositif selon la revendication 2, **caractérisé**
**en ce qu'**il existe exactement un diaphragme maculaire (MB) qui est disposé dans le plan (F‴) conjugué au fond (F) dans le trajet de faisceau d'éclairage (1.1), et le repère de fixation (FM) est une ouverture ponctuelle dans un centre de surface (FMP) du diaphragme maculaire (MB).

6. Dispositif selon la revendication 5, **caractérisé**
**en ce qu'**une barrette (ST) partiellement transparente, orientée radialement par rapport au centre de surface (FMP), est adjacente au diaphragme maculaire (MB) et permet de recouvrir la papille (P) au niveau du fond (F), de sorte que l'intensité de rayonnement d'une représentation de la papille (P) peut être adaptée à une plage dynamique du capteur d'image numérique (2), qui est conçue pour l'intensité de rayonnement d'une représentation des zones du fond (F) entourant la papille (P).

7. Dispositif selon la revendication 6, **caractérisé**
**en ce qu'**il existe un autre diaphragme partiellement transparent avec une transparence réglable pour recouvrir la papille (P).

8. Dispositif selon la revendication 6 ou 7, **caractérisé**
**en ce que** le diaphragme maculaire (MB) peut être déplacé dans le plan (F‴) conjugué au fond (F) dans le trajet de faisceau d'éclairage (1.1), son centre de surface (FMP) restant situé à l'intérieur du diaphragme de champ (FB), ce qui permet d'éclairer différentes zones sélectionnées du fond (F) par le champ lumineux (LF) et d'enregistrer des séquences d'images des différentes zones sélectionnées du fond (F).

9. Dispositif selon la revendication 1, **caractérisé**
**en ce qu'**exactement un diaphragme maculaire (MB) est formé sur le diaphragme de champ (FB) de telle sorte que le repère de fixation (FM) se trouve à l'intérieur du diaphragme de champ (FB) de manière adjacente à un bord intérieur, et le diaphragme de champ (FB) peut tourner autour d'un axe optique du trajet de faisceau d'éclairage (1.1), grâce à quoi différentes zones sélectionnées du fond (F) sont éclairées par le champ lumineux (LF) et des séquences d'images des différentes zones sélectionnées du fond (F) peuvent être prises.

10. Dispositif selon la revendication 1, **caractérisé**
**en ce qu'**exactement quatre diaphragmes maculaires (MB) sont formés sur le diaphragme de champ (FB) en étant opposés par paires en diagonale de telle sorte que le repère de fixation (FM) respectif se trouve à l'intérieur du diaphragme de champ (FB) en étant adjacent à un bord intérieur, grâce à quoi différentes zones prédéterminées du fond (F) sont éclairées par le champ lumineux (LF) et que des séquences d'images des différentes zones prédéterminées du fond (F) peuvent être prises.

11. Dispositif selon la revendication 1, **caractérisé**
**en ce que** l'unité d'éclairage (3) est formée, pour la réalisation de différentes structures d'éclairage, par un agencement adaptatif et structurable de sources lumineuses dans le plan (AP") conjugué à la pupille de l'œil (AP), qui peuvent être activées et désactivées localement, spectralement et temporellement de manière quelconque et séparée les unes des autres et/ou dont l'intensité peut être modulée.

12. Dispositif selon la revendication 1, **caractérisé**
**en ce qu'**une image du diaphragme de champ (FB) se trouvant dans le trajet de faisceau d'éclairage (1.1) sur le capteur d'image numérique (2) présente une section transversale plus petite qu'une surface de réception (2.1) du capteur d'image numérique (2), et la répartition de la luminosité dans une zone de différence (2.2) qui se forme est utilisée pour déterminer une répartition de la lumière parasite avec laquelle les images des séquences d'images peuvent être corrigées.

13. Procédé d'analyse de la fonction endothéliale vasculaire rétinienne des vaisseaux de la rétine au fond (F) d'un oeil (A), comprenant les étapes de procédé :
- réglage d'une caméra de fond d'oeil (1) sur l'œil (A),
- au moyen d'une unité d'éclairage,
production de lumière de mesure pour éclairer les vaisseaux de la rétine au niveau du fond (F) et de lumière scintillante pour stimuler les vaisseaux de la rétine au niveau du fond (F) pendant une phase de stimulation (SP),
- au moyen de la caméra de fond d'oeil,
production d'une séquence d'images d'une zone du fond (F) pendant une phase de base (BP), au moins une phase de stimulation (SP) et au moins une postphase (NP),
- mesure des diamètres des segments vasculaires sélectionnés des vaisseaux de la rétine dans les images de la séquence d'images générée, en fonction du lieu et du temps,
- correction de mouvement pour les segments de vaisseaux mesurés, dans laquelle chaque segment de vaisseau est attribué à un emplacement sur le fond (F) avec une correction de mouvement,
- formation de signaux de diamètre (D(t,x,y)) qui représentent les diamètres des vaisseaux mesurés en fonction du temps et de l'emplacement du segment de vaisseau sélectionné à chaque fois,
- déduction des paramètres vasculaires à partir des signaux de diamètre (D(t,x,y)), qui décrivent chacun la fonction endothéliale du segment vasculaire respectivement sélectionné,
où
l'éclairage et la stimulation de la macula (M) au niveau du fond (F) sont empêchés, en ce qu'au moins un diaphragme maculaire (MB), sur lequel se trouve un repère de fixation (FM), est représenté de manière nette sur le fond (F) et l'œil (A) est orienté par rapport à l'un des diaphragmes maculaires (MB) par fixation sur le repère de fixation (FM) de l'une des au moins un diaphragme maculaire (MB), de telle sorte que la macula (M) est recouverte par une représentation de l'un des diaphragmes maculaires (MB).

14. Procédé selon la revendication 13, **caractérisé**
**en ce que** des séquences d'images de zones différentes sélectionnées du fond (F) sont prises successivement, chaque fois après qu'exactement un diaphragme maculaire (MB) représenté sur le fond (F) a été déplacé dans une autre position, de sorte que sa représentation sur le fond (F) a été déplacée et que l'œil (A) a suivi en fixant le repère de fixation (FM).

15. Procédé selon la revendication 14, **caractérisé**
**en ce que** les paramètres de position des positions du diaphragme maculaire (MB) sont enregistrés et peuvent être rappelés et réglés pour des mesures répétées et consécutives.

16. Procédé selon la revendication 13, **caractérisé**
**en ce que**, en variante, des séquences d'images de zones différentes prédéfinies du fond (F) sont prises, tandis que quatre diaphragmes maculaires (MB), qui sont disposés diagonalement en vis-à-vis, sont représentés sur le fond (F) et que, à chaque fois, l'œil (A) a fixé successivement un autre des repères de fixation (FM).

17. Procédé selon la revendication 13, **caractérisé**
**en ce que** la durée de la phase de base (BP), de la phase de stimulation (SP) et de la postphase (NP) et les paramètres de la lumière de mesure et de la lumière de scintillement sont réglés indépendamment les uns des autres et sont enregistrés en étant affectés à un patient et à un programme d'examen, de sorte qu'ils peuvent être appelés et réglés à nouveau en tant que jeu de paramètres pour des examens répétés et comparatifs.

18. Procédé selon la revendication 13, **caractérisé**
**en ce que** l'on détermine comme l'un des paramètres vasculaires dérivés le maximum de dilatation par la lumière scintillante (FDₘₐₓ) et on l'édite dans un protocole de mesure conjointement avec une représentation graphique de signaux de diamètre moyens (D(t,x,y)) qui sont formés respectivement pour les segments vasculaires artériels sélectionnés et pour les segments vasculaires veineux sélectionnés à partir des signaux de diamètre (D(t,x,y)) formés à cet effet.

19. Procédé selon la revendication 18, **caractérisé**
**en ce que** le maximum de dilatation par la lumière scintillante (FDₘₐₓ) est attribué dans une image de cartographie comme imagerie fonctionnelle de la fonction endothéliale au segment vasculaire respectivement associé en étant codé en couleur, une dilatation vasculaire manquante ou une dilatation vasculaire en dessous d'une valeur seuil prédéfinie étant marquée en rouge et une dilatation vasculaire au-dessus d'une valeur seuil, qui correspond à une fonction vasculaire saine, étant marquée en vert sur le segment vasculaire associé et l'image de cartographie étant éditée comme résultat d'examen graphique.
